# EUROPEAN PATENT SPECIFICATION

(11) **EP 4 081 229 B1**
(45) Date of publication and mention of the grant of the patent: **25.02.2026**
(21) Application number: 20845483.5
(22) Date of filing: 23.12.2020
(51) Int. Cl.: A61K 35/66, A61K 35/741, A61K 35/744, A61K 38/44, A61P 1/00, A61P 17/00, A61P 29/00, A61P 31/04, A23L 33/135, C12N 9/06

(54) **CONTROL OF HISTAMINE TO INCREASE FEEDING EFFICIENCY AND TREAT OR PREVENT PREVENTING AND/OR TREATING CLOSTRIDIAL DERMATITIS AND/OR CLOSTRIDIAL ENTERIC DISEASE USING BREVIBACTERIUM AUREUM, BREVIBACTERIUM SEDIMINIS, AND/OR BREVIBACTERIUM EPIDERMIDIS**
HISTAMINKONTROLLE ZUR ERHÖHUNG DER FÜTTERUNGSEFFIZIENZ UND ZUR BEHANDLUNG ODER VORBEUGUNG VON CLOSTRIDIALE DERMATITIS UND/ODER CLOSTRIDIALE ENTERISCHE ERKRANKUNGEN MIT BREVIBACTERIUM AUREUM, BREVIBACTERIUM SEDIMINIS UND/ODER BREVIBACTERIUM EPIDERMIDIS
RÉGULATION DE L'HISTAMINE POUR AUGMENTER L'EFFICACITÉ ALIMENTAIRE ET TRAITER OU PRÉVENIR LA PRÉVENTION ET/OU LE TRAITEMENT DE LA DERMATITE À CLOSTRIDIE ET/OU DE LA MALADIE ENTÉRIQUE À CLOSTRIDIE À L'AIDE DE BREVIBACTERIUM AUREUM, BREVIBACTERIUM SEDIMINIS ET/OU BREVIBACTERIUM EPIDERMIDIS

(30) Priority: 24.12.2019 US 201962953299 P
(43) Date of publication of application: 02.11.2022
(73) Proprietor: Iowa State University Research Foundation, Inc., Ames, IA 50010 (US)
(72) Inventor: LYTE, Mark, Ames, IA 50011 (US)
(74) Representative: FRKelly
(86) International application number: PCT/US2020/066783
(87) International publication number: WO 2021/133877

(56) References cited:
- EP-A1- 1 964 570
- EP-A1- 1 964 570
- WO-A1-2017/105267
- WO-A2-2015/025336
- WO-A2-2015/025336
- TW-B- I 592 483
- US-A1- 2019 142 877
- US-A1- 2019 142 877
- US-B2- 8 586 053
- US-B2- 8 586 053
- ANONYMOUS: "Certain microbes may reduce allergy-like reactions in many people -- ScienceDaily", 16 April 2019 (2019-04-16), XP093068401, Retrieved from the Internet <URL:https%3A%2F%2Fwww.sciencedaily.com%2Freleases%2F2019%2F04%2F190416081404.htm> [retrieved on 20230728]
- DELAPLAIN PATRICK T. ET AL: "Effects of artificially introduced Enterococcus faecalis strains in experimental necrotizing enterocolitis", PLOS ONE, vol. 14, no. 11, 1 November 2019 (2019-11-01), pages e0216762, XP093068489, Retrieved from the Internet <URL:https://www.ncbi.nlm.nih.gov/pmc/articles/PMC6824573/pdf/pone.0216762.pdf> DOI: 10.1371/journal.pone.0216762
- HANCHI HASNA ET AL: "The Genus Enterococcus: Between Probiotic Potential and Safety Concerns-An Update", FRONTIERS IN MICROBIOLOGY, vol. 9, 3 August 2018 (2018-08-03), XP093014248, DOI: 10.3389/fmicb.2018.01791
- M. ROYAN: "The Use of Enterococci as Probiotics in Poultry", 1 December 2018 (2018-12-01), XP093068497, Retrieved from the Internet <URL:https://ijas.rasht.iau.ir/article_544713_0152c665c9f24e8c99895f3540b0fde0.pdf> [retrieved on 20230728]
- PARK J.W. ET AL: "Effect of dietary supplementation with a probiotic (Enterococcus faecium) on production performance, excreta microflora, ammonia emission, and nutrient utilization in ISA brown laying hens", POULTRY SCIENCE, vol. 95, no. 12, 1 December 2016 (2016-12-01), Oxford, pages 2829 - 2835, XP093068499, ISSN: 0032-5791, Retrieved from the Internet <URL:https://pdf.sciencedirectassets.com/776861/1-s2.0-S0032579119X60423/1-s2.0-S0032579119317912/main.pdf?X-Amz-Security-Token=IQoJb3JpZ2luX2VjEN3//////////wEaCXVzLWVhc3QtMSJGMEQCIF/eiAY9Xo2tqwyFpkXHeKt68rndJrDTAmd2C2iDMv6wAiA8Q7y+inP1Q+y5KpOE8z2ABu+2ghr/eMnIVBYlzZYztCqzBQh2EAUaDDA1OTAwMzU0Njg2NSIMRCAvZ> DOI: 10.3382/ps/pew241
- SMITH ALEXANDER B. ET AL: "Enterococci enhance Clostridioides difficile pathogenesis", NATURE, vol. 611, no. 7937, 24 November 2022 (2022-11-24), London, pages 780 - 786, XP093068507, ISSN: 0028-0836, Retrieved from the Internet <URL:https://www.ncbi.nlm.nih.gov/pmc/articles/PMC9691601/pdf/nihms-1845454.pdf> DOI: 10.1038/s41586-022-05438-x
- TITTARELLI FABRIZIA ET AL: "Biogenic amines producing and degrading bacteria: A snapshot from raw ewes' cheese", LWT- FOOD SCIENCE AND TECHNOLOGY, vol. 101, March 2019 (2019-03-01), pages 1 - 9, XP085567796, ISSN: 0023-6438, DOI: 10.1016/J.LWT.2018.11.030
- ANONYMOUS: "Certain microbes may reduce allergy-like reactions in many people -- ScienceDaily", 16 April 2019 (2019-04-16), XP93068401, Retrieved from the Internet <URL:https%3A%2F%2Fwww.sciencedaily.com%2Freleases%2F2019%2F04%2F190416081404.htm> [retrieved on 20230728]
- ANONYMOUS: "Certain microbes may reduce allergy-like reactions in many people -- ScienceDaily", 16 April 2019 (2019-04-16), XP093068401, Retrieved from the Internet <URL:https%3A%2F%2Fwww.sciencedaily.com%2Freleases%2F2019%2F04%2F190416081404.htm> [retrieved on 20230728]
- DELAPLAIN PATRICK T. ET AL: "Effects of artificially introduced Enterococcus faecalis strains in experimental necrotizing enterocolitis", vol. 14, no. 11, 1 November 2019 (2019-11-01), pages e0216762, XP093068489, Retrieved from the Internet <URL:https://www.ncbi.nlm.nih.gov/pmc/articles/PMC6824573/pdf/pone.0216762.pdf> DOI: 10.1371/journal.pone.0216762
- HANCHI HASNA ET AL: "The Genus Enterococcus: Between Probiotic Potential and Safety Concerns-An Update", FRONTIERS IN MICROBIOLOGY, vol. 9, 3 August 2018 (2018-08-03), XP093014248, DOI: 10.3389/fmicb.2018.01791
- M. ROYAN: "The Use of Enterococci as Probiotics in Poultry", 1 December 2018 (2018-12-01), XP093068497, Retrieved from the Internet <URL:https://ijas.rasht.iau.ir/article_544713_0152c665c9f24e8c99895f3540b0fde0.pdf> [retrieved on 20230728]
- PARK J.W. ET AL: "Effect of dietary supplementation with a probiotic (Enterococcus faecium) on production performance, excreta microflora, ammonia emission, and nutrient utilization in ISA brown laying hens", vol. 95, no. 12, 1 December 2016 (2016-12-01), Oxford, pages 2829 - 2835, XP093068499, ISSN: 0032-5791, Retrieved from the Internet <URL:https://pdf.sciencedirectassets.com/776861/1-s2.0-S0032579119X60423/1-s2.0-S0032579119317912/main.pdf?X-Amz-Security-Token=IQoJb3JpZ2luX2VjEN3//////////wEaCXVzLWVhc3QtMSJGMEQCIF/eiAY9Xo2tqwyFpkXHeKt68rndJrDTAmd2C2iDMv6wAiA8Q7y+inP1Q+y5KpOE8z2ABu+2ghr/eMnIVBYlzZYztCqzBQh2EAUaDDA1OTAwMzU0Njg2NSIMRCAvZ> DOI: 10.3382/ps/pew241
- SMITH ALEXANDER B. ET AL: "Enterococci enhance Clostridioides difficile pathogenesis", vol. 611, no. 7937, 24 November 2022 (2022-11-24), London, pages 780 - 786, XP093068507, ISSN: 0028-0836, Retrieved from the Internet <URL:https://www.ncbi.nlm.nih.gov/pmc/articles/PMC9691601/pdf/nihms-1845454.pdf> DOI: 10.1038/s41586-022-05438-x
- TITTARELLI FABRIZIA ET AL: "Biogenic amines producing and degrading bacteria: A snapshot from raw ewes' cheese", LWT- FOOD SCIENCE AND TECHNOLOGY, vol. 101, March 2019 (2019-03-01), pages 1 - 9, XP085567796, ISSN: 0023-6438, DOI: 10.1016/J.LWT.2018.11.030
- AISHATH NAILA ET AL: "Histamine Degradation by Diamine Oxidase, Lactobacillus and Vergibacillus halodonitrificans Nai18", JOURNAL OF FOOD PROCESSING & TECHNOLOGY, vol. 03, no. 06, 23 June 2012 (2012-06-23), XP055100638, DOI: 10.4172/2157-7110.1000158
- ANAST JUSTIN M. ET AL: "Brevibacterium from Austrian hard cheese harbor a putative histamine catabolism pathway and a plasmid for adaptation to the cheese environment", SCIENTIFIC REPORTS, vol. 9, no. 1, 16 April 2019 (2019-04-16), XP055783853, Retrieved from the Internet <URL:http://www.nature.com/articles/s41598-019-42525-y> DOI: 10.1038/s41598-019-42525-y
- RAHIMI S. ET AL: "Effect of direct-fed microbials on performance and Clostridium perfringens colonization of turkey poults", POULTRY SCIENCE, vol. 90, no. 11, 1 November 2011 (2011-11-01), Oxford, pages 2656 - 2662, XP055783875, ISSN: 0032-5791, DOI: 10.3382/ps.2011-01342
- ALIAKBARPOUR H. R. ET AL: "The Bacillus subtilis and Lactic Acid Bacteria Probiotics Influences Intestinal Mucin Gene Expression, Histomorphology and Growth Performance in Broilers", ASIAN-AUSTRALASIAN JOURNAL OF ANIMAL SCIENCES., vol. 25, no. 9, 22 August 2012 (2012-08-22), KR, pages 1285 - 1293, XP055783886, ISSN: 1011-2367, Retrieved from the Internet <URL:https://www.ncbi.nlm.nih.gov/pmc/articles/PMC4092943/pdf/ajas-25-9-1285-11.pdf> DOI: 10.5713/ajas.2012.12110
- ANONYMOUS: "Specifications | PrimaLac", 15 March 2016 (2016-03-15), XP055783887, Retrieved from the Internet <URL:https://web.archive.org/web/20160315200347/https://primalac.com/primalac/specifications/> [retrieved on 20210310]
- N LIU ET AL: "Effect of Broccoli Residues Fermented with Probiotics on the Growth Performance and Health Status of Broilers Challenged with Clostridium Perfringens", REVISTA BRASILEIRA DE CIÊNCIA AVÍCOLA, vol. 20, no. 4, 1 October 2018 (2018-10-01), pages 625 - 632, XP055747310, ISSN: 1516-635X, DOI: 10.1590/1806-9061-2018-0741
- KAO LI ET AL: "Beneficial effects of the commercial lactic acid bacteria product, Vigiis 101, on gastric mucosa and intestinal bacterial flora in rats", JOURNAL OF MICROBIOLOGY, IMMUNOLOGY AND INFECTION, ELSEVIER, AMSTERDAM, NL, vol. 53, no. 2, 23 June 2018 (2018-06-23), pages 266 - 273, XP086119341, ISSN: 1684-1182, [retrieved on 20180623], DOI: 10.1016/J.JMII.2018.06.002
- SEKIGUCHI YOSHINORI ET AL: "A thermostable histamine oxidase from Arthrobacter crystallopoietes KAIT-B-007", JOURNAL OF BIOSCIENCE AND BIOENGINEERING, vol. 97, no. 2, 1 January 2004 (2004-01-01), NL, pages 104 - 110, XP055784395, ISSN: 1389-1723, DOI: 10.1016/S1389-1723(04)70176-0
- LI J ET AL: "Comparative study between probiotic bacterium Arthrobacter XE-7 and chloramphenicol on protection of Penaeus chinensis post-larvae from pathogenic vibrios", AQUACULTURE, ELSEVIER, AMSTERDAM, NL, vol. 253, no. 1-4, 31 March 2006 (2006-03-31), pages 140 - 147, XP027902971, ISSN: 0044-8486, [retrieved on 20060331]
- LI JIQIU ET AL: "Immune Responses and Resistance against Vibrio parahaemolyticus Induced by Probiotic Bacterium Arthrobacter XE-7 in Pacific White Shrimp, Litopenaeus vannamei", JOURNAL OF THE WORLD AQUACULTURE SOCIETY, 1 August 2008 (2008-08-01), Malden, USA, pages 477 - 489, XP055784466, Retrieved from the Internet <URL:https://onlinelibrary.wiley.com/doi/epdf/10.1111/j.1749-7345.2008.00188.x> [retrieved on 20210311], DOI: 10.1111/j.1749-7345.2008.00188.x
- GOBBETTI ET AL: "Arthrobacter", ENCYCLOPEDIA OF FOOD MICROBIOLOGY (SECOND EDITION) 2014, PAGES 69-76, 1 January 2014 (2014-01-01), pages 69 - 76, XP055784398, Retrieved from the Internet <URL:https://www.sciencedirect.com/science/article/pii/B9780123847300000094?via%3Dihub>
- YUANYUAN WU ET AL: "Effects of supplementation on the growth performance and gut health of broiler chickens with -induced necrotic enteritis", JOURNAL OF ANIMAL SCIENCE AND BIOTECHNOLOGY, BIOMED CENTRAL LTD, LONDON, UK, vol. 9, no. 1, 25 January 2018 (2018-01-25), pages 1 - 14, XP021252922, DOI: 10.1186/S40104-017-0220-2
- DELPHINE L. CALY ET AL: "Alternatives to Antibiotics to Prevent Necrotic Enteritis in Broiler Chickens: A Microbiologist's Perspective", FRONTIERS IN MICROBIOLOGY, vol. 6, 1 December 2015 (2015-12-01), XP055498685, DOI: 10.3389/fmicb.2015.01336

## Description

### FIELD OF THE INVENTION

The present invention relates to probiotic compositions for use in a method of preventing or treating clostridial dermatitis or clostridial enteric disease, and methods of improving feeding efficiency.

### BACKGROUND OF THE INVENTION

There is ongoing understanding on the role of commensal microbiota and their influence on behavior, health, and disease susceptibility. Commensal or commensalism refers to a relationship between organisms that feed from each other in a way that neither benefits nor harms the relationship of the microbiota. Infections in farm production animals, companion animals, aquaculture and humans can cause significant damage or behavioral changes to the animals/humans and cause significant economic harm in the instance of animal producers. Various infections and inflammatory conditions within the gut of these species, namely farm production animals, is a multi-billion dollar problem within the animal food production industry.

According to a 2012 Animal and Plant Health Inspection Service USDA report, 42.3 percent of turkey farms have problems with clostridial dermatitis (CD). CD can cause swelling, fluid accumulation, and vesicles on the subcutaneous breast region. Turkeys with this condition usually die; the 2012 report highlights the mortality rate of 4-17% due to CD outbreaks. CD is a disease of turkeys having significant economic impact on turkey producers across various geographic regions. Its prevalence and severity continues to increase and it has been identified as the most significant disease facing the turkey industry. The significant economic losses that turkey farmers have to bear result from bird mortality at marketable age, increased condemnation rates, and expensive medication costs for treatment. Economic losses ascribed to CD were projected to be $1.31 per affected bird. CD is a major health problem in commercial poultry raised on deep litter systems as are customary in US farms, however no known mechanisms other than the involvement of *Clostridial* spp. is known and more importantly no known effective treatments are known.

Similar to the deep litter systems used in turkey farms, the use of hardwood shavings as litter in chicken coops has been found to correlate with higher incidence of cellulitis. Moreover, survival of poultry pathogens in built-up litter is known to contribute to persistence and carry-over of related diseases to subsequent flocks in the barns. There has been little study into the impact of environmental risk factors on the incidence of CD in turkeys or the incident in cellulitis in chickens. However, the problems in the industry, causing reductions in avian farm production animals have gone unsolved.

However, bacterial infection are not the only way inflammatory or behavioral conditions can develop. As many of the microbiota, especially *Clostridial* species, are normal members of the gut microbiota (as described herein as commensal bacteria) in farm production animals as well as humans. As such, inflammation or behavioral conditions may result from dysregulation of the normal microbial diversity (such as by stress or other factor) thereby allowing those members of the microbiota to either become greater in number (abundance) or produce more histamine. Accordingly, there is a need for preventing/treating not only infections but also controlling effects of members of the microbiota that can cause increases in histamine production.

Anast Justin M. et al. disclose "Brevibacterium from Austrian hard cheese harbor a putative histamine catabolism pathway and a plasmid for adaptation to the cheese environment", in SCIENTIFIC REPORTS vol. 9, no. 1, 16 April 2019 DOI: 10.1038/s41598-019-42525-y.

US8586053B2 discloses a phytopercolate comprising *Brevibacterium* for use in the treatment of ulcerative colitis, and inflammatory bowel disease.

EP1964570A1 discloses the treatment of allergies including food allergies by means of a composition comprising *Brevibacterium* spp.

WO2015/025336A2 discloses a probiotic composition comprising the isolated bacterial strain *Brevibacterium casei* AP9 MCC0012 exhibiting bile hydrolase activity.

Accordingly, it is an objective of the invention to formulate probiotic compositions and/or histamine degrading enzymes capable reducing histamine production, such as by consuming histamine in the gut of a subject, including farm production animals, companion animals, aquaculture, and humans, to promote gut health any psychological well-being.

Another objective is to provide compositions for use in methods for prophylaxis and/or treatment of subjects in need of prevention and/or treatment of conditions caused by histamine overproduction, including for example clostridial dermatitis and clostridial enterocolitis.

Other objects, advantages and features of the present invention will become apparent from the following specification taken in conjunction with the accompanying figures.

### BRIEF SUMMARY OF THE INVENTION

The invention is defined in the claims.

Provided is a probiotic composition for use in a method of preventing and/or treating clostridial dermatitis and/or clostridial enteric disease in a subject comprising: histamine-degrading bacteria, wherein the bacteria comprise *Brevibacterium aureum, Brevibacterium sediminis,* and/or *Brevibacterium epidermidis*; wherein said composition controls histamine production from a commensal microbiota in the subject.

Also provided is a non-therapeutic method of increasing feeding efficiency of a subject comprising administering: a probiotic composition comprising histamine-degrading bacteria, wherein the bacteria comprise *Brevibacterium aureum, Brevibacterium sediminis,* and/or *Brevibacterium epidermidis;* wherein said composition controls histamine production in the subject.

An advantage of the invention is the ability to reduce histamine overproduction which is known to cause various inflammatory and behavioral conditions in farm production animals, companion animals, aquaculture, and humans. Beneficially, the use of probiotics able to degrade histamine can prevent and/or treat the farm production animals, companion animals, aquaculture and human species having conditions associated with histamine overproduction.

The foregoing summary is illustrative only and is not intended to be in any way limiting. In addition to the illustrative aspects, embodiments, and features described above, further aspects, embodiments, and features will become apparent by reference to the drawings and the following detailed description. Accordingly, the drawings and detailed description are to be regarded as illustrative in nature and not restrictive.

### BRIEF DESCRIPTION OF THE DRAWINGS

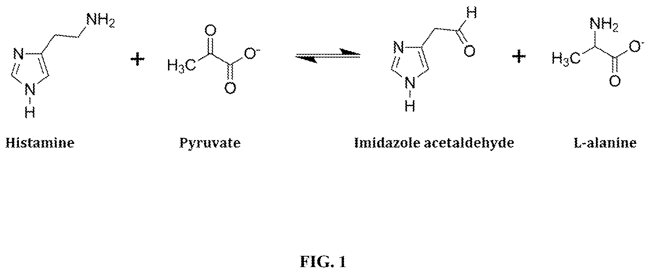

Figure 1 shows the chemical reaction of a histamine-pyruvate aminotransferase transferring an amine from histamine to pyruvate and in the process generating alanine.

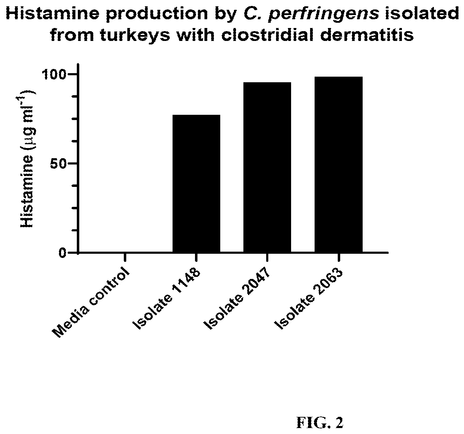

Figure 2 (reference only) is a graph depicting that three *Clostridium perfringens* isolated from turkeys with clostridial dermatitis are able to produce large amounts of histamine in vitro.

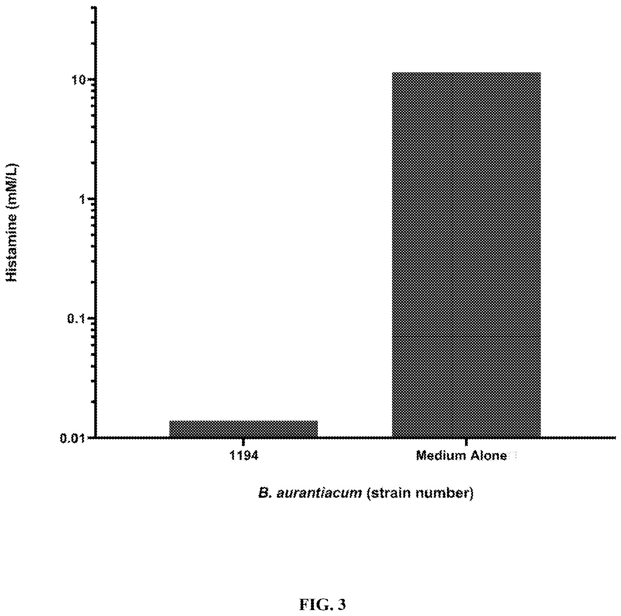

Figure 3 is a graph depicting that *Brevibacterium* spp. can degrade histamine.

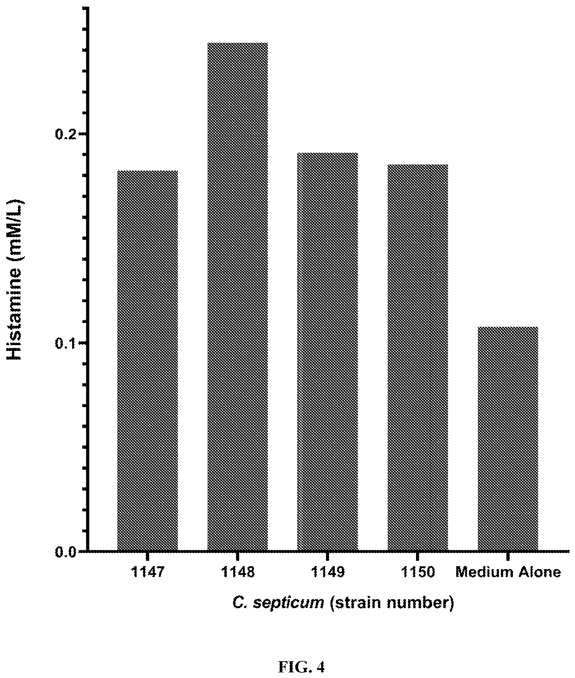

Figure 4 (reference only) shows a graph depicting *C*. *septicum* producing histamine.

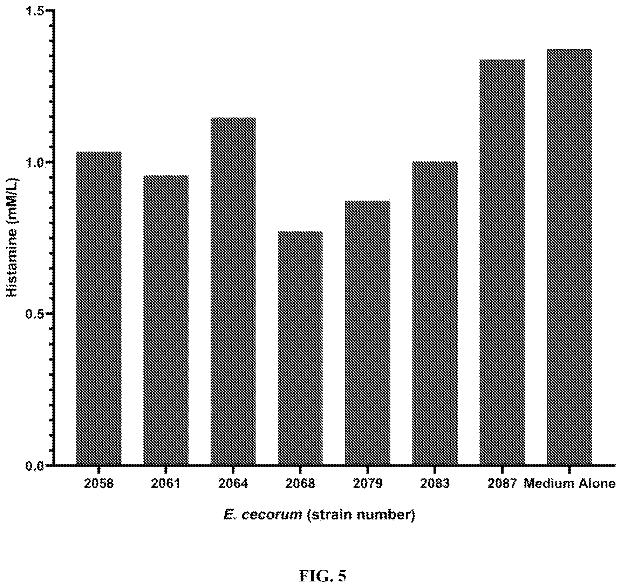

Figure 5 (reference only) shows a graph depicting *Enterococcus cecorum* degrading histamine.

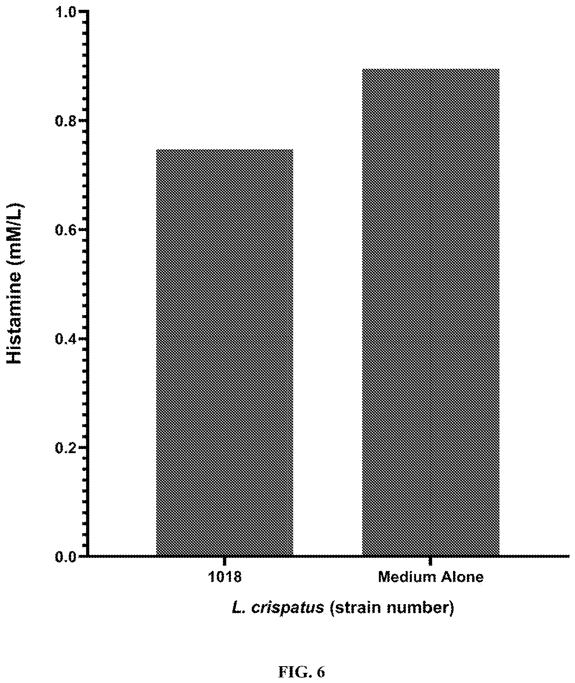

Figure 6 (reference only) shows a graph depicting *Lactobacillus crispatus* degrading histamine.

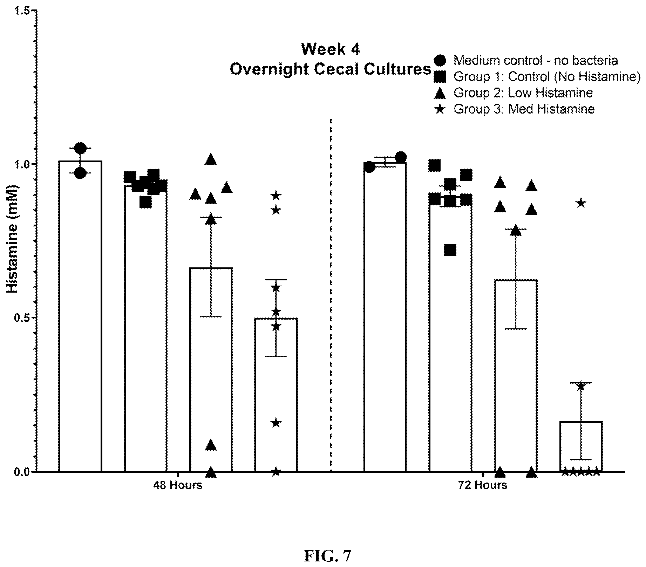

Figure 7 shows an example of histamine degradation in an overnight cecal cultures for chickens of 4 weeks of age enrolled as part of Histamine Feeding Trial #1. Cultures were grown in 10mL of 1/3^{rd} BHI with 1mM histamine at 41.5°C in a microaerophilic atmospheric environment. Samples were collected at 48 and 72 hours for ultra-high-performance liquid chromatography (UHPLC) analysis of histamine content.

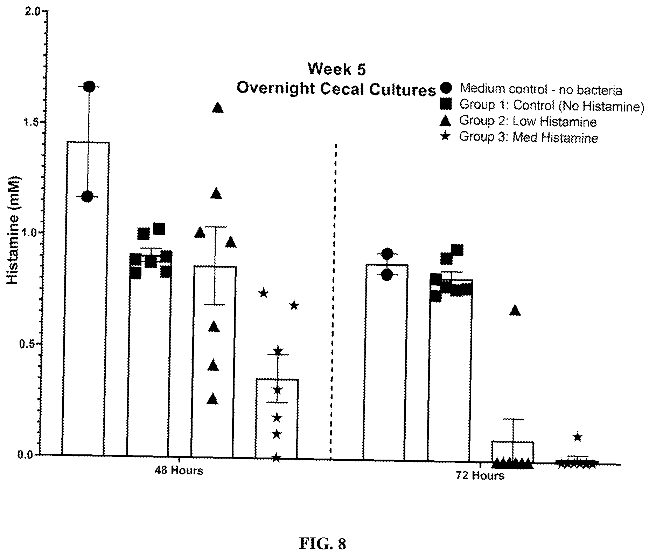

Figure 8 shows an example of histamine degradation in an overnight cecal cultures for chickens of 5 weeks of age enrolled as part of Histamine Feeding Trial #1. Cultures were grown in 10mL of 1/3^{rd} BHI with 1mM histamine at 41.5°C in a microaerophilic atmospheric environment. Sample was collected at 48 and 72 hours for UHPLC analysis of histamine content.

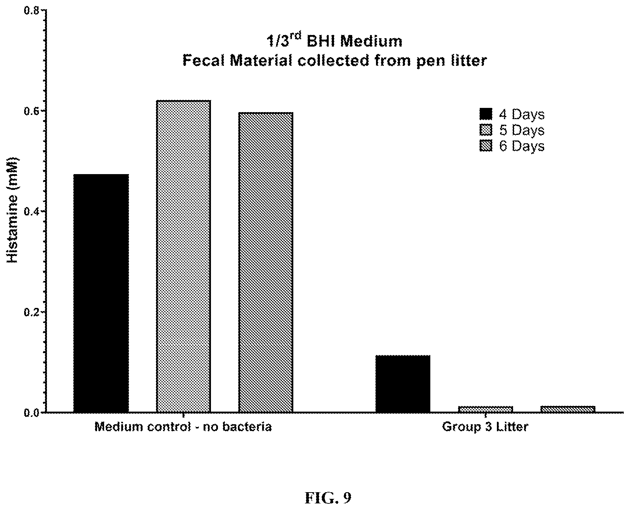

Figure 9 shows an example of histamine degradation in fecal matter collected from Group #3 pen litter for chickens of 6 weeks of age from Histamine Feeding Trial #2 and cultured in 1/3^{rd} BHI supplemented with 1mM histamine in a microaerophilic atmospheric environment at 41.5°C. Cultures were sampled at 4, 5, and 6 days after initial inoculation for UHPLC analysis of histamine content. As substantial histamine degradation was shown, samples were then removed from the culture tubes and subjected to isolation using selective medium for the identification of histamine-degrading bacteria by culture.

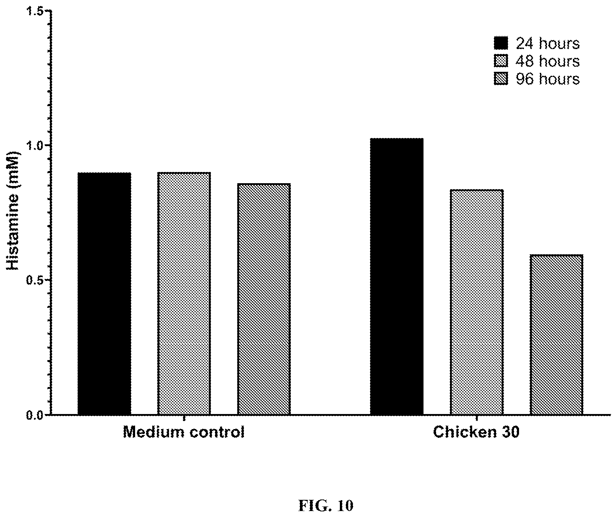

Figure 10 shows an example of histamine levels in cecal matter of a chicken from Group #2, Histamine Feeding Trial #2 that was fed for 5 weeks was collected and cultured in 1/3^{rd} BHI supplemented with 1mM histamine in a microaerophilic atmospheric environment at 41.5°C. Cultures were sampled at 1, 2, and 4 days after initial inoculation for UHPLC analysis of histamine content. As substantial histamine degradation was shown, samples were then removed from the culture tubes and subjected to isolation using selective medium for the identification of histamine-degrading bacteria by culture.

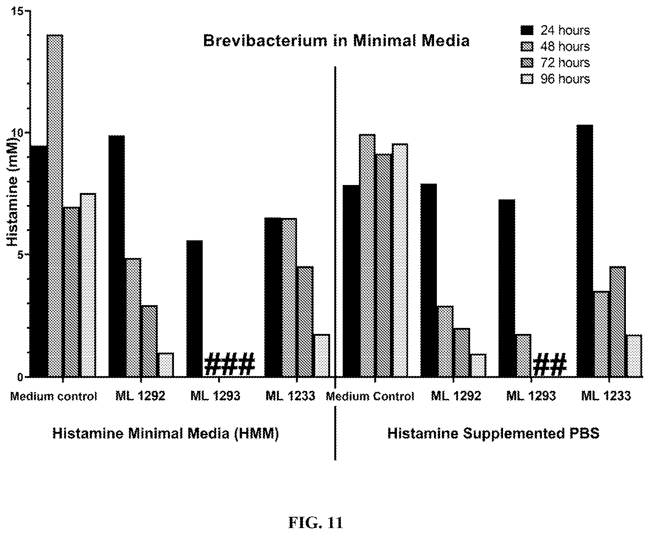

Figure 11 shows an example of histamine being degraded by *Brevibacterium* spp. grown over 96 hours in minimal mediums supplemented with histamine. Samples were incubated aerobically at 30° C and received constant agitation during the growth process. At indicated times samples were removed and assayed for histamine using UHPLC technique. "#" symbol in figure indicates no detectable values for histamine. ML# designates strain number.

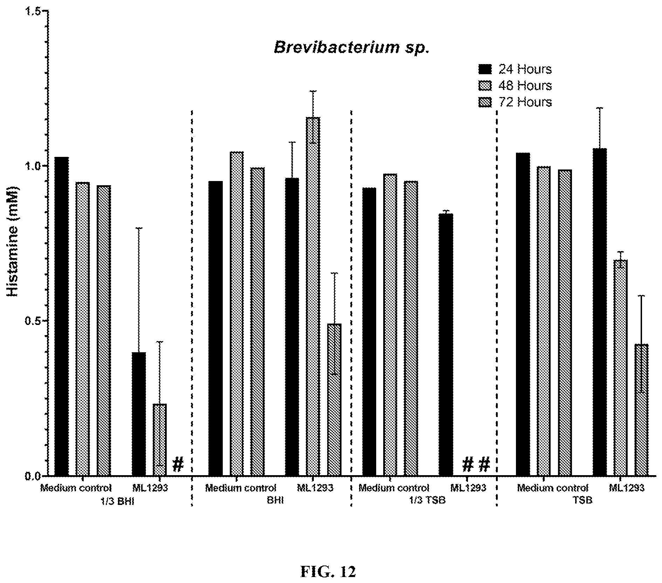

Figure 12 shows an example of histamine being degraded by a *Brevibacterium* sp. grown over 72 hours in nutrient and depleted nutrient mediums supplemented with histamine (1mM). Samples were incubated aerobically at 30° C and received constant agitation during the growth process. Cultures were sampled at indicated times for processing of histamine concentration by UHPLC analysis. "#" symbol in figure indicates no detectable values for histamine. ML# designates strain number.

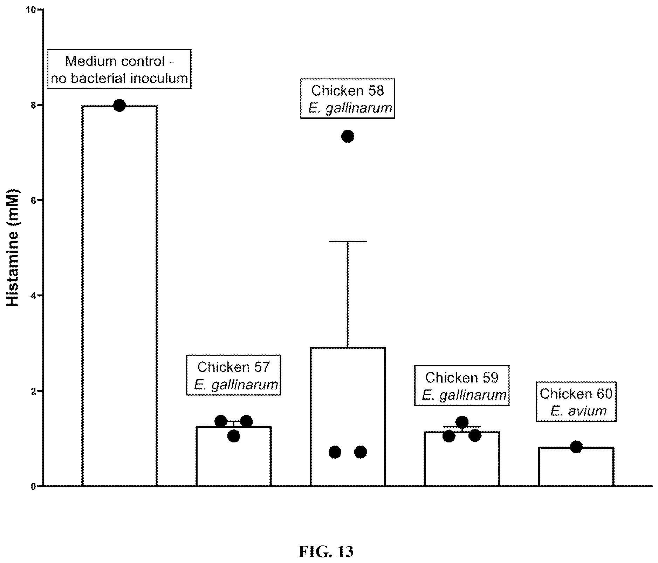

Figure 13 (reference only) shows an example of *Enterococcus* spp. isolated from chickens fed a histamine-containing diet and subsequently identified using MALDI-TOF technologies which were inoculated into 1/3^{rd} BHI medium and the amount of histamine culture. Samples were incubated in a microaerophilic environment at 41.5° C for 120 hours before sampling for UHPLC analysis of histamine content.

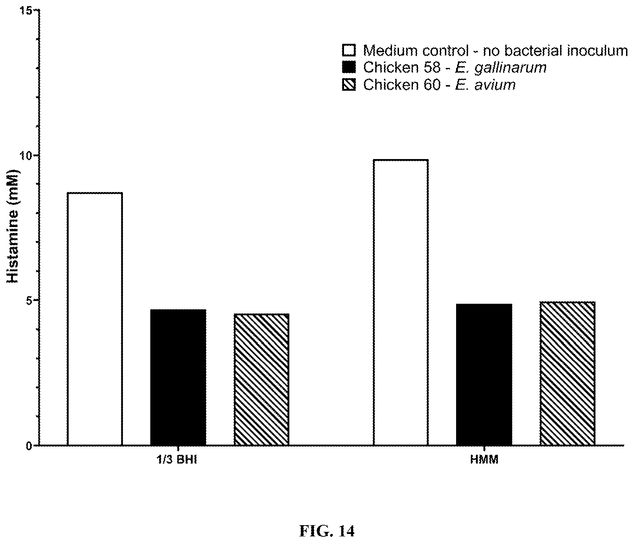

Figure 14 (reference only) shows an example of *Enterococcus* spp. isolated from chickens fed a histamine-containing diet and subsequently identified using MALDI-TOF technologies which were inoculated into 1/3^{rd} BHI or Histamine Minimal Medium (HMM) which contained 10mM histamine and the amount of histamine post culture. Samples were incubated in a microaerophilic environment at 41.5° C for 120 hours before sampling for UHPLC analysis of histamine content.

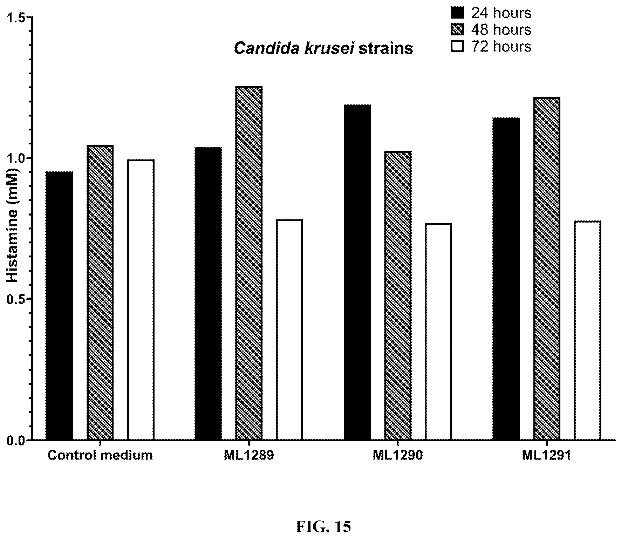

Figure 15 (reference only) shows examples of three different strains of the yeast *Candida krusei* were incubated aerobically in BHI supplemented with 1mM of histamine at 41.5° C with rocking at a speed of 20 RPM and a platform tilt of 15-degree for 72 hours. At indicated times samples were withdrawn and analyzed for histamine using UHPLC technique.

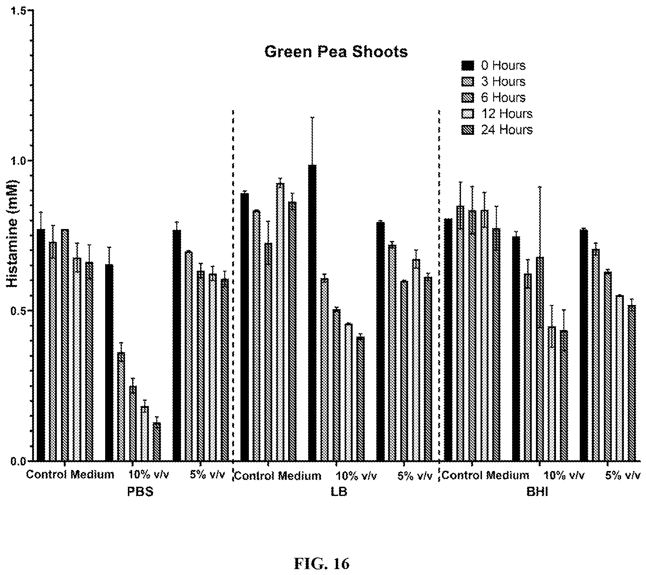

Figure 16 (reference only) shows an example of histamine being degraded by material extracted from green pea shoots grown in a soil-free see tray in the dark and harvested at 10 days before being ground into pulp. Extracted plant material was filter-sterilized by passage through a 0.22µm syringe filter and was then inoculated into histamine-supplemented media (BHI, LB, and PBS) at a v/v ratio of 10% or less before being incubated over a 24 hours' time range. At indicated times, samples were withdrawn from tubes and assayed for histamine content using UHPLC technique.

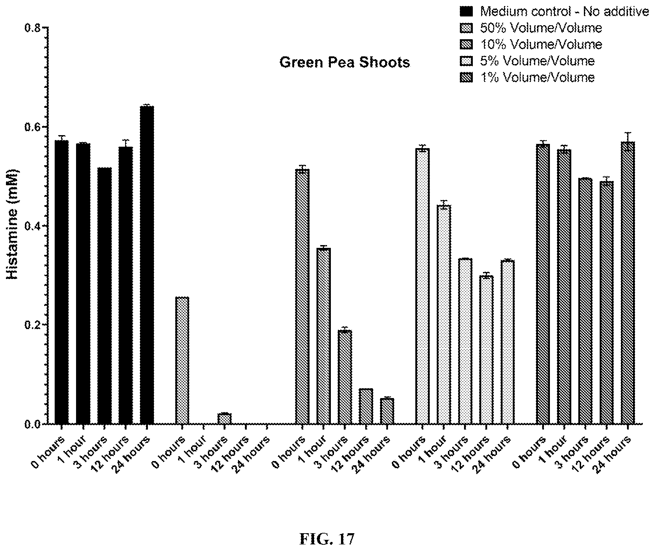

Figure 17 (reference only) shows an example of histamine being degraded by material extracted from green pea shoots grown in a soil-free see tray in the dark and harvested at 10 days before being ground into pulp. Extracted plant material was filter-sterilized by passage through a 0.22µm syringe filter and was then inoculated into histamine supplemented medium (PBS) at a v/v ratio of 50% or less before being incubated over a 24 hours' time range. At indicated times, samples were withdrawn from tubes and assayed for histamine content using UHPLC technique.

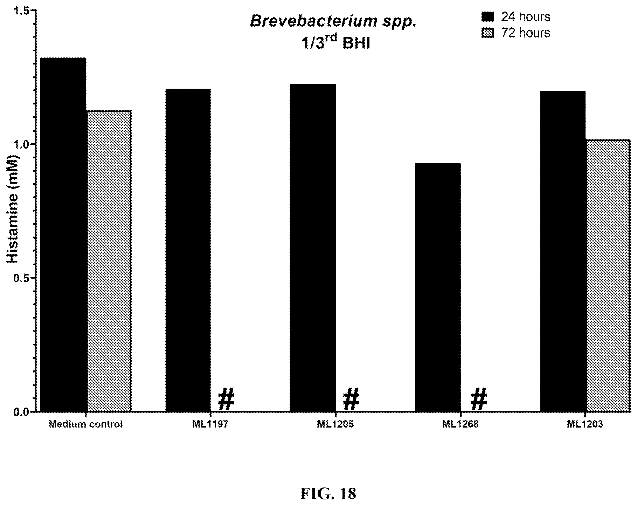

Figure 18 shows an example of *Brevibacterium* spp. grown in 1/3^{rd} BHI at 37°C over 72 hours in an aerobic environment on a rocking platform set at a 15-degree tilt and 20 RPM speed to provide constant agitation during the growth process. Cultures were sampled at indicated times for processing of histamine concentration by UHPLC analysis. "#" symbol in figure indicates no detectable values for histamine. ML# designates strain number.

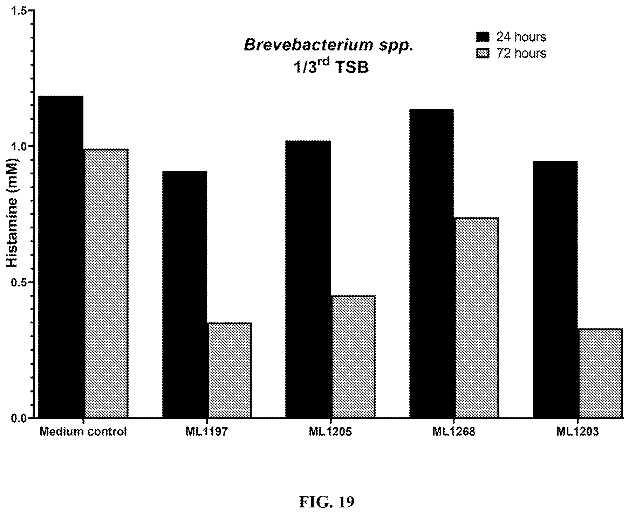

Figure 19 shows an example of *Brevibacterium* spp. grown in 1/3^{rd} TSB at 37°C for 72 hours in an aerobic environment on a rocking platform set at a 15-degree tilt and 20 RPM speed to provide constant agitation during the growth process. Cultures were sampled at indicated times for processing of histamine concentration by UHPLC analysis. ML# designates strain number.

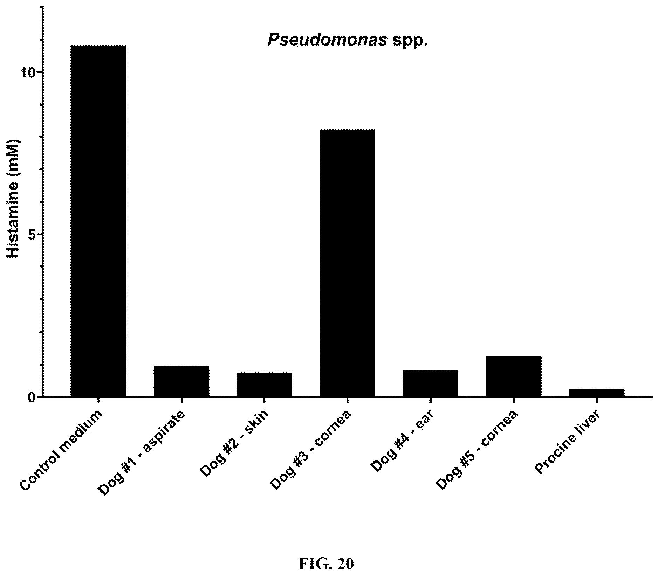

Figure 20 (reference only) shows an example of *Pseudomonas aeruginosa* isolated from various animal species grown in HMM supplemented with 10mM histamine for 24 hours at 37°C in an aerobic environment. Cultures were sampled and processed for quantification of histamine by UHPLC analysis.

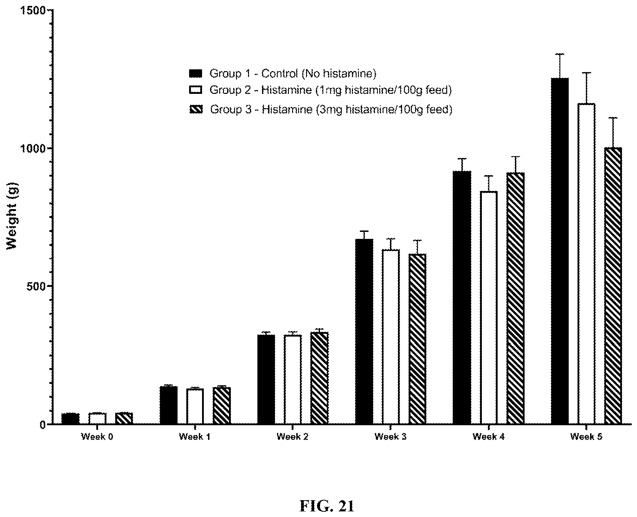

Figure 21 (reference only) shows a graphical example of the weekly bodyweight of chickens fed different histamine diets from Histamine Feeding Trial #1.

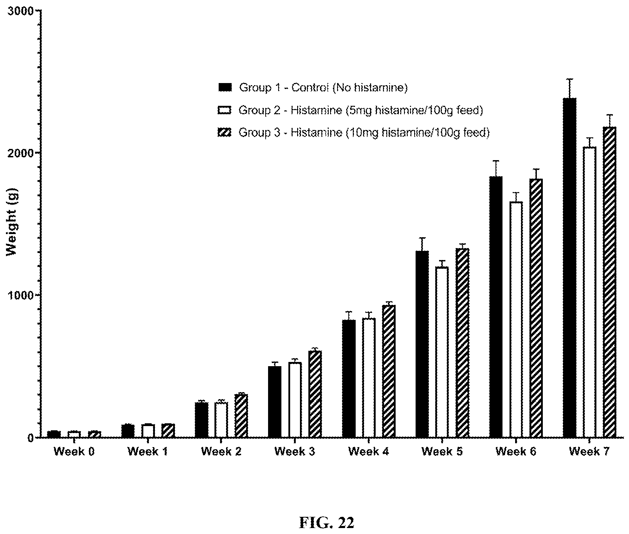

Figure 22 (reference only) shows a graphical example of the weekly bodyweight of chickens fed different histamine diets from Histamine Feeding Trail #2.

Reference to various embodiments does not limit the scope of the invention. Figures represented herein are not limitations to the various embodiments according to the invention and are presented for exemplary illustration of the invention.

### DETAILED DESCRIPTION OF THE INVENTION

The present invention is defined in the claims. The embodiments are not limited to particular methods and compositions depicted herein, which can vary and may be understood by skilled artisans. It is further to be understood that all terminology used herein is for the purpose of describing particular embodiments only and is not intended to be limiting in any manner or scope. For example, as used in this specification and the appended claims, the singular forms "a," "an" and "the" can include plural referents unless the content clearly indicates otherwise. Further, all units, prefixes, and symbols may be denoted in its SI accepted form.

The phrase "and/or," when used between elements in a list, is intended to mean either (1) that only a single listed element is present, or (2) that more than one element of the list is present. For example, "A, B, and/or C" indicates that the selection may be A alone; B alone; C alone; A and B; A and C; B and C; or A, B, and C. The phrase "and/or" may be used interchangeably with "at least one of" or "one or more of" the elements in a list.

In order to provide a clear and consistent understanding of the specification and the claims, including the scope given to such terms, the following definitions are provided. Units, prefixes, and symbols may be denoted in their SI accepted form.

The term "about," as used herein, refers to variations in size, distance or any other types of measurements that can be resulted from inherent heterogeneous nature of the measured objects and imprecise nature of the measurements itself. The term "about" also encompasses variation in the numerical quantity that can occur, for example, through typical measuring and liquid handling procedures used for making media and reagents; through inadvertent error in these procedures; through differences in the manufacture, source, or purity of the ingredients used to make the compositions or carry out the methods, and moreover may modify the typical measurements referenced herein, and the like.

As used herein, the term "administering" refers to providing the probiotic or synbiotic composition to the subject. In one embodiment, the subject is provided the probiotic or synbiotic composition internally, and/or the administration of histamine-degrading enzyme either coated to protect activity, or alone, or as part of a mash, by a method or route which results in at least partial localization of the compound or composition to the gut or other hollow organ (e.g. oral cavity) such that a desired effect is produced. A composition described herein can be administered to the subject by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

In another embodiment, the probiotic or synbiotic compositions may be provided "topically" to the skin. As used herein, the term "topically" includes providing the compositions externally or by a shallow injection under the skin.

As used herein, an "effective amount" or "therapeutically effective amount" refers to the amount of the probiotic composition that is sufficient to prevent, treat, reduce and/or ameliorate the symptoms and/or underlying causes of a disorder or disease. In an exemplary aspect, an "effective amount" or "therapeutically effective amount" refers to the amount of probiotic that is sufficient to prevent, inhibit, and/or treat clostridial dermatitis, clostridial enteric disease, and/or gut inflammation in the gut of the subject, including farm production animals, companion animals, aquaculture, and humans.

Also, as used herein, the term "gut" refers to the gastrointestinal tract as well as the liver, spleen, pancreas, omentum, and other organs served by the blood supply to and from the gut.

The term "microbiome", as used herein, refers to a population of microorganisms from a particular environment, including the environment of the body or a part of the body. The term is interchangeably used to address the population of microorganisms itself (sometimes referred to as the microbiota), as well as the collective genomes of the microorganisms that reside in the particular environment. The term "environment," as used herein, refers to all surrounding circumstances, conditions, or influences to which a population of microorganisms is exposed. The term is intended to include environments in a subject, such as a bird. Specifically, the term "intestinal microbiota", as used herein, refers to the population of microorganisms inhabiting the gastrointestinal tract. The term was previously referred to as the intestinal flora. The term "skin microbiota", as used herein, refers to the population of microorganisms inhabiting the skin.

"Microorganism" refers to an organism or microbe of microscopic, submicroscopic, or ultramicroscopic size that typically consists of a single cell. Examples of microorganisms include bacteria, viruses, parasites, fungi, certain algae, and protozoa. The term "microbial" indicates pertaining to, or characteristic of a microorganism.

"Non-pathogenic bacteria" refers to bacteria that under normal conditions do not cause a disease or harmful responses in a healthy host. In some embodiments, non-pathogenic bacteria are commensal bacteria. Examples of non-pathogenic bacteria include, but are not limited to *Bacillus* spp., *Bacteroides* spp., *Bifidobacterium* spp., *Brevibacterium* spp., *Clostridium* spp., *Enterococcus* spp., *Escherichia coli, Lactobacillus* spp., *Lactococcus* spp., *Saccharomyces* spp., and *Staphylococcus* spp. Naturally pathogenic bacteria may be genetically engineered to provide reduced or eliminate pathogenicity according to standard methods in the art. Non-pathogenic bacteria may be genetically engineered to enhance or improve desired biological properties, e.g., survivability. Non-pathogenic bacteria and/or yeast may be genetically engineered to provide probiotic properties. Bacteria and/or yeast may be genetically engineered to be non-pathogenic. Without being limited to a particular mechanism of the invention, probiotics differ in their ability to produce neurochemicals in the gut of a subject. Non-pathogenic bacteria may be used for probiotic or synbiotic compositions used to treat subjects, while either pathogenic or non-pathogenic bacteria may be used for production of dopamine in a bioreactor or media. Pathogenicity, or virulence, of *E. faecium* may be defined as in the European Food Safety Authority, Scientific Opinion on the safety and efficacy of Oralin® (Enterococcus faecium) as a feed additive for calves for rearing, piglets, chickens for fattening, turkeys for fattening and dogs, EFSA Journal 2014;12(6):3727, 19 pp. (doi:10.2903/j.efsa.2014.3727) in section 2.1.1.

The term "population", as used herein, refers to a plurality of individual organisms, in the context of this invention, the term refers in particular to a collection of organisms of diverse taxonomic affiliation, in particular bacteria.

"Probiotic" is used to refer to live, non-pathogenic microorganisms, e.g., bacteria or fungi which may confer health benefits to a host organism that contains an appropriate amount of the microorganism. In some embodiments, the host organism is a farm production animal, companion animal, aquaculture and/or human. Some species, strains, and/or subtypes of non-pathogenic bacteria are currently recognized as probiotics. Examples of probiotics include, but are not limited to, *Candida* spp., *Debaryomyces* spp., *Debaryomyces* spp., *Enterococcus* spp., *Kluyveromyces* spp., *Kluyveromyces* spp., *Saccharomyces* spp., *Yarrowia* spp., *Bifidobacteria* spp., *Escherichia coli, Vagococcus* spp., *Carnobacterium* spp., *Melissococcus* spp. and *Lactobacillus* spp., e.g., *Candida humilis, Debaryomyces hansenii, Debaryomyces occidentalis, Kluyveromyces lactis, Kluyveromyces lodderae, Kluyveromyces marxianus, Saccharomyces cerevisiae, Saccharomyces boulardii, Yarrowia lipolytica, Bifidobacterium bifidum, Enterococcus faecium, Enterococcus faecalis, Enterococcus hirae, Enterococcus casseliflavus, Enterococcus gallinarum, Escherichia coli* strain Nissle, *Lactobacillus acidophilus, Lactobacillus bulgaricus, Lactobacillus paracasei, Lactobacillus plantarum, Vagococcus fluvaialis* (Dinleyici et al., 2014; U.S. Pat. No. 5,589,168; U.S. Pat. No. 6,203,797; U.S. Pat. No. 6,835,376). A probiotic may also be a variant or a mutant strain of bacterium (Arthur et al., 2012; Cuevas-Ramos et al., 2010; Olier et al., 2012; Nougayrede et al., 2006).

As used herein, the term "synbiotic" means a mixture of probiotic and prebiotics which may confer health benefits to a host organism. The term "prebiotic" as used herein means a cofactor that may improve the survivability of the probiotic microorganisms or induce the growth of activity of beneficial microorganisms such as bacteria or fungi. Therefore, a synbiotic composition is a probiotic composition comprising a probiotic and a cofactor, and, as such, probiotic and synbiotic are used interchangeably herein.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C2-C12 alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

Methods of treatment are not claimed. By "treatment", "treat," "prevention," "prevent" or the like of an adverse condition, infection and/or disease is meant delaying or preventing the onset of such a condition, infection and/or disease, reversing, alleviating, ameliorating, inhibiting, slowing down or stopping the progression, aggravation or deterioration the progression or severity of a condition associated with such an adverse condition. In one embodiment, at least one symptom of an adverse condition is alleviated by at least 1%, at least 5%, at least 10%, at least 20%, at least 30%, at least 40%, or at least 50%.

The term "sufficient amount of time," as used herein, refers to the time it takes for a compound, probiotic strain, or the like which is effective for producing some desired effect in at least a sub-population of cells.

The term "E.C." or "EC" as used herein, refers to the Enzyme Commission number. The E.C. number does not refer to specific enzymes, but to enzyme-catalyzed reactions. Therefore, they represent a group of enzymes that catalyze the same reaction. The E.C. may have up to four numbers which each successive number representing a group of enzymes with a more specific function and/or substrate.

### Histamine

As used herein, the term "histamine" is a small organic molecule C₅H₉N₃ that is a biogenic amine that participates in neural, immune and other general physiological activities. Specifically, histamine is a compound released by cells, primarily mast cells, in response to injury, infection, allergy and/or inflammatory reactions. Diet may also contribute to the amount of histamine present in the body as a reduction in histamine in the diet has been shown to be linked to the reduction of atopic dermatitis symptoms in humans. Histamine is generally formed across all species, both eukaryotic and prokaryotic, by decarboxylation of histidine though a histidine decarboxylase (E.C. 4.1.1.22).

Histamine, through its various receptors has been implicated in a wide range of physiological and behavioral processes. Through their receptors, histamines primarily act as immune mediators and neurotransmitters. The compound generally causes dilation of capillaries, contraction of smooth muscles, and stimulation of gastric acid secretion. Histamines may also affect behavior, such as locomotor activity like scratching, sleep-wake cycles and wakefulness, cognition, and feed intake.

There are currently four known histamine receptors (NCBI MeSH ID: D011968), abbreviated H₁-H₄ or H₁R-H₄R. These receptors are characterized by their binding affinity to various histamine agonists and show some organ and cell specificity. This affinity and specificity modulate the various physiological and behavioral responses to histamine.

H₁ receptors (NCBI MeSH ID: D011969) generally work through the Gαq/11 pathway activating the inositol phosphate/diacylglycerol second messenger system to increase intracellular Ca²⁺. This increase results in smooth muscle contraction, increased vascular permeability, hormone and cytokine release, induces the production of chemokines, prostacyclin and platelet activating factor, and cerebral glyconeogenesis. Hence, almost all immediate hypersensitivity reactions and symptoms observed in the skin, such as erythema, pruritus, and edema, may be elicited through histamine.

H₁ receptors are primarily immune related receptors. They may enhance Th1 and Th2 immune responses by releasing IL-4 and IL-13 to differentiate Th0 cells as well as inhibiting INF-γ and causing Th2 migration to the lungs. Their activation may also lead to B cell proliferation.

H₁ receptors, like H₃ receptors below, have also been implicated in feeding behavior and energy homeostasis. Antipsychotic drugs may act as H₁ antagonists and have been shown to cause significant weight gain in humans. Hence, systemic histamine may affect feeding behavior to such a degree as to cause disease in an organism and returning it to normal levels will return the behaviors to normal. The gut-brain axis consists of bidirectional communication between the central and the enteric nervous system, linking emotional and cognitive centers of the brain with peripheral intestinal functions. Therefore, a change in the histamine level in the gut may work through the gut-brain axis to change these behaviors.

H₁ receptors are also expressed in dermal dendritic cells and keratinocytes in skin tissue. Here H₁ receptors, in addition to differentiation and recruitment of immune cells, increase IL-31 production. This affects pruritus and skin barrier functions in allergic dermatitis. Therefore, H₁ receptors may also induce the various symptoms related with skin diseases, such as atopic dermatitis, and the augmentation of the immune system with histamine may increase the immune response to skin diseases, such as Clostridial dermatitis, to exacerbate the dermatitis. As such, it may be desirable to reduce histamine in a subject to control symptoms.

H₂ receptors (NCBI MeSH ID: D011970), unlike H₁ receptors, are expressed on a wide range of cells and tissues, including B and T cells, dendritic cells, gastricparietal cells, smooth muscle cells, brain, and cardiac tissues. They are coupled to Gαs stimulated adenylyl cyclases and can induce airway mucus production, vascular permeability, inotropic and chronotropic effects of heart muscle, relaxation of smooth muscle, and secretion of gastric acid. Impairment of H₂ receptors have been associated with impaired immune functions, gastric acid secretion, and certain cognitive functions, including hippocampal potentiation impairments, which may affect learning and memory, and nociception abnormalities.

H₃ receptors (NCBI MeSH ID: D018100) are expressed exclusively in neurons and coupled to Gαi/o. Here they act as inhibitory auto-receptors and regulate the release of several neurotransmitters in the central and peripheral nervous systems. They are important for homeostatic regulation of energy levels, sleep-wake cycles, cognition, and inflammation. Impaired H₃ receptors and/or antagonists may impair locomotion and effect behavior. This may result in metabolic syndrome characterized by late onset obesity due to hyperphagia and increased leptin and insulin levels. H₃ antagonists have been shown to reverse diet-induced obesity. H₃ receptor antagonists may also be myocardial ischemic arrhythmias, cognition disorders, and insomnia.

Taken together, the stimulation of H₁ and H₃ receptors are at least partially responsible for overall feeding behavior and effect it in such a way that may cause disease when perturbed. When perturbed by a loss of histamine, a subject may alter their normal behavior and exhibit hyperphagia which may result in late onset obesity. Therefore, increasing systemic histamine will decrease feed intake and prevent obesity. However, in healthy subjects the increased histamine may cause undesired weight loss.

H₄ receptors (NCBI MeSH ID: D000074040) are Gα/io coupled and are also expressed on a variety of cells, including immune cells, including eosinophiles, T cells, dendritic cells and mast cells, as well as the spleen, intestinal epithelia, lungs, synovial tissue, central nervous system, sensory neurons, and cancer tissues. H₄ receptors mediates the pro-inflammatory responses of histamine in both an autocrine and paracrine manner. This causes an increased migration of eosinophils, mast cell activation, the expression of pro-inflammatory cytokines and chemokines. H₄ receptor activation, therefore, results in an increase in chemotaxis of mast cells and their accumulation at sites of allergic responses; primes mast cells for allergen-induced activation; and Ca²⁺ dependent mast cell activation. As the recruitment of mast cells may lead to the amplification of an immune response, it may be desirable to reduce the histamine in a subject to prevent chronic inflammation.

H₄ receptors also increase bone marrow-derived basophils following antigen stimulation. This basophil regulation has been associated with allergic dermatitis by inducing chemotaxis of the basophils. Therefore, reducing histamine, such as by providing a probiotic containing a histamine degrading enzyme or organism, may treat and/or prevent dermatitis by lowering the stimulation of the immune cells through H₁ and H₄ receptors.

H₄ receptors are also preferentially expressed in Th2 cells over native T and Th1 cells and may be involved in the pathogenesis of allergy and inflammation. Therefore, reduction of histamine may also prevent the pathogenesis related to chronic allergen exposure and inflammation similar to the treatment of dermatitis.

Taken together, reducing histamine, such as by providing a probiotic containing a histamine degrading enzyme or organism, may treat and/or prevent diseases related to inflammation, such as dermatitis, behavior, and digestion in a subject.

Breakdown of histamine has multiple pathways (KEGG Pathway ID: ko00340) but is typically performed by a class of enzymes belonging to oxidoreductases which act on the CH-NH2 group of the doner compound (E.C. 1.4). More preferably, enzymes belonging to E.C. 1.4.3.22. For example, the amine oxidase (copper containing) (AOC) family of enzymes: AOC1, AOC2, and AOC3, also known in the art as diamine oxidase (DAO), histaminase, histamine oxidase, amiloride-sensitive amine oxidase (copper-containing), amine oxidase copper domain-containing protein, amiloride-binding protein 1 (ABP1), and kidney amine oxidase.

In other organism, such as those lacking an immune system, histamine may still be used in addition to produced. For example, while bacteria may produce histamine, they may also use histamine as a carbon source. Some strains of bacteria, for example strains of *Brevibacterium,* will survive in a minimal media supplemented with histamine as the only carbon source.

### Subjects

The probiotic compositions for use described herein are particularly suitable for use with farm production animals, companion animals, aquaculture, and humans.

Farm production animals include for example, poultry, turkeys, and any avian species. Farm production animals further include ruminants and non-ruminants. Companion animals include for example, dogs, cats, and horses. Aquaculture includes all species.

The probiotic compositions for use described herein are particularly suitable for use in avian species. All birds are part of the Class Aves (Phylum Chordata and Subphylum Vertebrata). Examples of suitable avian species that may be treated with the probiotic compositions described herein include, but are not limited to, chickens, ducks, geese, turkeys, guinea fowl, ostriches, pigeons, quails, and pheasants. Examples of avian farm production animals most often include chickens and turkeys.

### Probiotic Compositions

The probiotic or synbiotic compositions comprise histamine-degrading bacteria, wherein the bacteria comprise *Brevibacterium aureum, Brevibacterium sediminis,* and/or *Brevibacterium epidermidis.* Said composition controls histamine production from a commensal microbiota in the subject.. The probiotic composition could alternatively administer a spore which then germinate into a histamine-degrader in the gut of the subject. In a further embodiment, the probiotic bacterial strain may further be modified to knock down or knock out histidine decarboxylase to prevent additional histamine from being produced by the probiotic strain.

In a further embodiment, the probiotic compositions may be provided as a mash in which the probiotic is incorporated and processed to release the enzymes contained within that can break down histamine in the gut. Exemplary enzymes include amine oxidase (copper containing). Examples of use of these enzymes in food preparations have been shown, such as the preparation of tuna soup containing very high levels of histamine, wherein the enzyme amine oxidase (copper containing) is shown to reduce histamine during the preparation of the soup. Without being limited to a particular mechanism of action, the activity of the amine oxidase (copper containing) was destroyed during the preparation in a final step of boiling, so no histamine degradation was ever proposed to occur following food consumption. Analogous studies employ such enzymes only during preparation of food and then destroy the activity prior to consumption. *See* Naila et al. Prediction of the amount and rate of histamine degradation by diamineoxidase (DAO). Food Chemistry 135:2650-2660, 2012.

In other embodiments, the compositions may further comprise of enzymes belonging to E.C. 1.4.3, such as amine oxidase (copper containing), facilitate the reaction RCH₂NH₂ (amine) + H₂O (water) to RCHO (aldehyde) + NH₃ (ammonia) + H₂O₂ (peroxide). Specifically, amine oxidase (copper containing) converts histamine (amine) into imidazole acetaldehyde (aldehyde), ammonia and peroxide. Many genera, including but not limited to bacteria, fungi, and plants, have been reported to possess this activity. Prominent examples of bacteria include strains of *Staphylococcus* including *S. xylosus, S. cronusus;* strains of *Brevibacterium* spp. including *B. linens*; *Arthrobacter crystallopoietes*; some *Lactobacilli* spp. as well as *Vergibacillus* spp., *Pseudomonas* spp., and *Escherichia* spp. The efficacy of these enzymes is maximized under neutral to alkaline conditions and oxygen must be available. It has been reported that amine oxidase (copper containing) can be directly added to certain fermented foods to achieve a decrease in histamine levels.

In other embodiments, the compositions may further contain enzymes which may degrade histamine by a dehydrogenase pathway. In this variant, histamine once again is utilized to generate imidazole acetaldehyde and ammonia. However, in contrast to oxidase pathways, dehydrogenase pathways frequently involve several steps including a transamination step and dehydrogenation step. This reaction also does not generate hydrogen peroxide. Consider the pathway recently described for *Pseudomonas putida.* In this pathway, several enzymes work in a concerted process. In particular HinC, a histamine-pyruvate aminotransferase (E.C. 2.6.1.58), transfers an amine from histamine to pyruvate and in the process generates alanine (Figure 1).

Imidazole acetaldehyde can be further processed by an aldehyde dehydrogenase (E.C. 1.2.1) enzyme like HinD to yield imidazole acetic acid. Pyruvate can also be regenerated from L-alanine, a process which liberates ammonia. Species notable for histamine degradation by dehydrogenase pathways include *Rhizobium, Nocardioides simplex* and *Natrinema gari.* Because this variant relies on transamination, it is conceivable that an abundance of a preferred amine bearing substrates may decrease the degradation of histamine whereas an abundance of pyruvate might enhance histamine degradation.

In an embodiment, probiotics may be grown in large fermenters and then pelleted, dried, and ground down. This processing destroys the viability of the organism yet preserves the activity of their enzymes to then allow the mash to be incorporated into the feed. Such methods must be done in accordance with accepted methods of preparation bacterial or fungal products for use in feeds.

In an embodiment, probiotics may be grown in large fermenters and then simply heat-inactivated with no further processing and then incorporated into the feed.

In an embodiment, the probiotics may not have a histidine decarboxylase (E.C. 4.1.1.22) enzyme or have been modified to knock down or knock out the function of histidine decarboxylase to prevent the probiotic from making histamine.

In yet a still further embodiment, the enzymes, such as in the form of purified enzyme (or one or more of the bacterial or yeast species listed herein) can be administered or incorporated into feed. Exemplary enzymes include amine oxidase (copper containing). In embodiments, enzymes can be coated to protect their activity during transit through the gut until they reach specific section of gut where the need for histamine degradation exists.

In further embodiments, for use in animal feeds, enzymes can be added "neat" but also can be coated to protect their activity. There are a number of coating strategies that are currently used to protect enzymes in feed. By way of nonlimiting example, different types of granulation or nanoparticle techniques may be used to coat the enzyme including providing a core or inner matrix to which the enzyme may be attached and then using one or more coatings to protect the enzyme from the heat, pressure, and moisture generated during feed manufacturing and then to allow delivery of a sufficient amount of the enzyme to the subject. Further see Hua S. (Advances in Oral Drug Delivery for Reginal Targeting in the Gastrointestinal Tract - Influence of Physiological, Pathophysiological and Pharmaceutical Factors, Front. Pharmacol, 2020, 11:524), Fenster, K., et al. (The Production and Delivery of Probiotics: A Review of a Practical Approach, Microorganisms 2019, 7, 83), and Govender, M., et al. (AAPS PharmSciTech, 2014, 15, 29) for delivering compounds to the gastrointestinal tract in general.

Histamine may be metabolized through at least four known mechanisms (as described in https://www.histaminintoleranz.ch/en/histaminosis_histaminemetabolism.html), including (1) Oxidative deamination through amine oxidase (copper containing), also known as DAO or histaminase, according to the following chemical equation: Histamine + H2O + O2 => (imidazole-4-yl)acetaldehyde + NH3 + H2O2; (2) Cyclical methylation through histamine N-methyltransferase (HNMT; E.C. 2.1.1.8), wherein the resulting product is N-methylhistamine (NMH); (3) Acetylation into acetylhistamine, which is the degradation pathway most important in microbial degradation by enzymes in E.C. 2.3.1; and (4) Hydroxylase into hydantoin propionic acid, wherein the enzymes can be present in microorganisms.

Various strains are suitable for inclusion in the probiotic compositions or to extract enzyme and the listing of the genera name and representative strains are not meant to limit in any way that other strains belonging to that genus may be suitable histamine degraders. The various strains can also be given as a probiotic (non-spore) or as a spore. The probiotic composition for use of the present invention comprises *Brevibacterium aureum, Brevibacterium sediminis,* and/or *Brevibacterium epidermidis.* Exemplary known but not claimed bacteria include: *Arthrobacter* spp., including for example *A. crystallopoietes*; *Bacillus* spp., including for example *B. subtilis, B. coagulans, B. lichenformis,* and *B. amyloliquefaciens; Brevibacterium* spp., including for example *B. linens; Micrococcus* spp.; *Rhizobium* spp.; *Enterococcus* spp., including for example *E. cecorum; Escherichia* spp., including for example *E. coli; Lactobacillus* spp., including for example *L. sakei, L. plantarum,* and *L. crispatus; Staphylococcus* spp., including for example *S. xylosus, S. caronusus; Agrobacterium* spp., including for example *A. tumefaciens; Vergibacillus* spp., including for example *V. halodenitrificans; Pseudomonas* spp., including for example *P*. *putida* or *P. aeruginosa; Candida* spp., including for example *C*. *krusei; Nocardioides* spp., including for example *N. simplex; Rummeliibacillus* spp., including for example *Rummeliibacillus stabekisii; Natrinema* spp., including for example *Natrinema gari; Debaryomyces* spp. (yeast), including for example *D. hansenii (fungi); Saccharomyces* spp. (yeast); *Yarrowia* spp., including for example *Y. lipolytica; Aspergillus* spp., including for example *Aspergillus oryzae* or *Aspergillus niger; Penicillium* spp., including for example *Penicillium italicum; Pinchia* spp., including for example *Pichina pastoris;* and *Gibberella* spp., including for example *Gibberella fujikuroi.*

The probiotic composition for use of the present invention comprises *Brevibacterium* spp: *Brevibacterium aureum, Brevibacterium sediminis,* and/or *Brevibacterium epidermidis.* Without being limited to a particular mechanism of action, the *Brevibacterium* spp. degrades histamine. *Brevibacterium* spp. are commercially available as they are used for inoculation of cheese rinds during ripening and/or can be established naturally from sources such as cheese rinds. They are most commonly found on surfaces of soft, semi-hard and hard cheeses. The strains are among the most abundant cheese rind bacteria, producing aroma compounds and having lipase and protease activity.

In some embodiments, the probiotic compositions include a therapeutically effective amount of at least one live, non-pathogenic probiotic strain, such as those listed above. The bacterial (or yeast) strains utilize histamine for its own survival and growth, thereby overcoming the excess histamine production by certain commensal microbiota.

In an embodiment, a therapeutically effective amount of the probiotic strain(s) is from about 10⁴ CFU to about 10¹⁴ CFU. In another embedment, a therapeutically effective amount of the histamine degrading enzyme is from about 10¹ histamine degrading units (HDU) to about 10¹⁰ HDU. In another embodiment, a therapeutically effect amount of the composition may be from about 10⁴ CFU to about 10¹⁴ CFU of a probiotic and from about 10¹ HDU to about 10¹⁰ HDU. As used herein, a HDU is the number of molecules of histamine that may be degraded.

The probiotic compositions may be administered to reach the gut as a lyophilized powder or in a tablet form. The lyophilized powder may be added to a liquid such as, but not limited to, water or food for ingestion. The tablet may be a chewable tablet. The probiotic may be administered live or heat inactivated dead cells, and in whole or in part. The parts of the probiotic may include cellular components, such as, but not limited to, the DNA or protein which are capable of rendering their beneficial effects. The probiotic compositions may be administered in any pharmaceutically acceptable formulation such as, but not limited to, a tablet or as part of a composition comprising the substrate and a pharmaceutically acceptable carrier.

Tablets and capsules for administration to the gut may be in unit dose form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tableting lubricant, for example magnesium stearate, talc, polyethylene glycol or silica; disintegrates for example potato starch, or acceptable wetting agents such as sodium lauryl sulphate. The tablets may be coated according to methods well known in normal pharmaceutical practice. Preferred coatings and encapsulation agents include pH-sensitive biocompatible polymers. By way of non-limiting example, these polymers may include poly-lactic acid, poly(lactic-co-glycolic acid), and chitosan. Other coatings, such as but not limited to hydroxypropyl methylcellulose phthalate or carboxymethyl high amylose starch, may also be used to enhance the delivery of probiotics to the gut. Alternatively, the coating may take advantage of the bacteria found in the gut and comprise a polymer that show degradation specificity for different regions of the gut. While biodegradable coatings may be used, they are not preferred because they can suffer from premature drug release or bursting due to their hydrophilicity and solubility in regions various regions of the gut, for example, in the upper gastrointestinal tract. The coatings may also contain more than a single compound or layer. An example of a multilayer coating may include a first protective coating which becomes degraded in the stomach, a second layer of a pH-dependent polymer coating which becomes degraded in the small intestine, and a third coating which the microorganism commensal to the colon may break down to release the probiotic to the colon. For examples, see Hua S. (2020), Fenster, K. (2019), and Govender, M. (2014).

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, Sorbian monooleate, polysorbate 80, or acacia; non-aqueous vehicles (which may include edible oils), for example almond oil, fractionated coconut oil, oily esters such as glycerin, propylene glycol, or ethyl alcohol; preservatives, for example methyl or propyl p-hydroxybenzoate or sorbic acid, and if desired conventional flavoring or coloring agents.

Topical compositions may be administered as live or heat inactivated dead cells, and in whole or in part. The parts of the probiotic may include cellular components, such as, but not limited to, the DNA or protein which are capable of rendering their beneficial effects. The probiotic compositions may be administered in any pharmaceutically acceptable formulation such as may be formulated as a lotion, shake lotion, cream, ointment, gel, foam, powder, solid, past, or tincture. In a further environment, the topical composition may further comprise a bandage, dressing, or combinations thereof.

In a further embodiment, a bandage or dressing is provided comprising the topical probiotic compositions described above. In yet further embodiments, a bandage or dressing is provided comprising the topical probiotic compositions described above, glycerol, and any combination thereof. In various aspects, a bandage or dressing is provided the major constituents of which includes a matrix and a topical probiotic composition. In various aspects, a bandage or dressing is provided the major constituents of which includes a matrix and a probiotic. In various aspects, a bandage or dressing is provided the major constituents of which includes a matrix and glycerol.

### Probiotic Compositions for Use in Methods of Prevention and/or Treatment

Methods of treatment and prevention of disease are not claimed. Any reference to such methods should be interpreted as "compositions for use in methods for treatment or prevention". Various conditions are suitable for prevention and/or treatment using the probiotic compositions. In an embodiment, the composition is provided for use in preventing and/or treating clostridial dermatitis and/or clostridial enteric disease in avian species Methods of increasing feed efficiency in avian species are also provided.

In an embodiment, the composition is provided for use in preventing and/or treating clostridial dermatitis and/or clostridial enteric disease in porcine species. Methods of increasing feed efficiency in porcine species are also provided.

In an embodiment, the composition is provided for use in preventing and/or treating clostridial dermatitis and/or clostridial enteric disease in ruminant species. Method of increasing feed efficiency are also provided.

In an embodiment, the composition is provided for use in preventing and/or treating clostridial dermatitis and/or clostridial enteric disease in aquaculture species. Methods of increasing feed efficiency are also provided.

In an embodiment, the composition is provided for use in preventing and/or treating clostridial dermatitis and/or clostridial enteric disease in humans.

The various infections, diseases and conditions for which prophylaxis and treatment according to the methods described herein are suited, may include a commensal microbiota, including *Clostridium* spp. causing an increase in histamine production in the subject. Exemplary *Clostridium* spp. include *Clostridium perfringens* and *Clostridium septicum,* including strains having Genbank accession no. KX674025, KX674031 and KX674026. Beneficially, the methods of preventing and/or treating using the probiotic compositions control histamine by providing a probiotic strain that degrades histamine, providing the subjects' immune system time and ability to respond.

The probiotic composition can be delivered to the subject. The delivering of the compositions can be provided in any conventional manner of dosing to birds. For example, the probiotic composition can be provided in a feed source, such as pellets.

The probiotic compositions for use control histamine production from a commensal microbiota or an infection in the subject being dosed the probiotic composition. These microorganisms include, but are not limited to, *Clostridium* spp., *Salmonella* spp., *Escherichia* spp., *Proteus* spp., *Morganella* spp., *Enterobacter* spp., *Klebsiella* spp., and *Lactobacillus* spp. In an embodiment, the commensal microbiota is a *Clostridium perfringens.* The delivering of the probiotic compositions can include administering to a subject with a *Clostridial* infection, or the administering to a subject as prophylaxis.

Without being limited to a particular mechanism of action, the probiotic composition for use in the method for prevention and/or treatment of a subject for clostridial dermatitis, clostridial enteric disease beneficially controls histamine production caused by a commensal microbiota, including for example *Clostridium perfringens* which have been shown to result in excess histamine production.

As one skilled in the art recognizes, there is a biochemical signaling in the gut-brain axis joining the microbiota, the alimentary tract (including the gastrointestinal tract) and the central nervous system. The gut-brain axis includes the microbiota in the alimentary tract, central nervous system, neuroendocrine and neuroimmune systems (e.g. hypothalamic-pituitary-adrenal axis), sympathetic and parasympathetic arms of the autonomic nervous system, and the gut microbiota. Beneficially, the probiotic compositions for use in methods of prophylaxis and treatment are suitable for adjuvant treatment of various pathologies of the gut. As described herein, a consequence of the dysregulated production of histamine is the effect on neuroimmune interactions driving inflammation and behavior.

The neuroimmune system includes the structures and processes involving the biochemical and electrophysiological interactions between the nervous system and immune system. These two systems are tightly integrated and have both local and systemic reflexes to restore homeostasis in response to injury and/or infection. The neuroimmune system is comprised primarily of glial cells and mast cells. As discussed above, histamine receptors, especially H₄, are expressed on mast cells and drive their activation. They key role of mast cells in inflammation and in the disruption of the blood-brain barrier may be achieved through the activation of the mast cells through these receptors. Mast cells also seem to participate in neuroinflammation both directly and through microglia stimulation. These two systems also communicate using a system of broad, common cytokines, growth factors, and neuropeptides, allowing for bidirectional communication. This cross talk permits the amplification of maladaptive inflammatory feedforward loops. Hence, the activation of mast cells through histamine receptors may contribute to the pathogenesis of such conditions such as headaches, autism, chronic fatigue syndrome, allergy, asthma, chronic coughing, autoimmunity, itch, and pain. As the gut-brain axis connects the gut to the brain, this is also linked to the neuroimmune system through the communication between the immune and nervous systems. Therefore, histamine production in the gut may influence behavioral changes through the gut-brain axis and mediated by mast cells. Accordingly, the methods described herein able to control histamine production by probiotic compositions beneficially control neuroimmune events, such as headaches, autism, chronic fatigue syndrome, allergy, autoimmunity, itch, and pain, in the subject.

The probiotic compositions for use comprise a therapeutically effective amount of at least one probiotic strain and/or histamine degrading enzyme. In an aspect, a therapeutically effective amount of the probiotic strain(s) in the composition includes from about 10⁴ CFU to about 10¹⁴ CFU, from about 10⁴ CFU to about 10¹² CFU, from about 10⁵ CFU to about 10¹¹ CFU, or from about 10⁵ CFU to about 10¹⁰ CFU.

In an embodiment, the method of administering is by oral administration. Oral administration can include various dosage forms as one skilled in the art will ascertain, including for example, tablets, capsules, aqueous or oily suspensions, solutions, emulsions, syrups or elixirs, dry products for reconstitution with water or other suitable vehicle before use.

Tablets and capsules for oral administration may be in unit dose form, and may contain conventional excipients such as binding agents, for example syrup, acacia, gelatin, sorbitol, or polyvinyl-pyrrolidone; fillers for example lactose, sugar, maize-starch, calcium phosphate, sorbitol or glycine; tableting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, polysorbate 80, or acacia; non-aqueous vehicles (which may include edible oils), for example almond oil, fractionated coconut oil, oily esters such as glycerin, propylene glycol, or ethyl alcohol; preservatives, for example methyl or propyl p-hydroxybenzoate or sorbic acid, and if desired conventional flavoring or coloring agents. Tablets and capsules may be formulated as a time release tablet or capsule to target different organs along the alimentary track. Similarly, the use of encapsulated and/or coated preparations can be employed, such as for the histamine degrading enzyme preparations.

In an embodiment, the method of administering is by topical administration. Topical administration can include various dosage forms as one skilled in the art will ascertain, including for example, lotions, creams, shake lotion, cream, ointment, gel, foam, powder, solid, paste, or tincture.

In another embodiment, the method of administering is by a bandage or dressing comprising the topical compositions described above. In a further embodiment, a bandage or dressing is provided comprising the topical probiotic compositions described above, glycerol, and any combination thereof. In various aspects, a bandage or dressing is provided the major constituents of which includes a matrix and a topical probiotic composition. In various aspects, a bandage or dressing is provided the major constituents of which includes a matrix and a probiotic. In various aspects, a bandage or dressing is provided the major constituents of which includes a matrix and glycerol.

The present invention is further illustrated by the following examples, which should not be considered as limiting in any way.

### EXAMPLES

### Example 1

As increased histamine has been associated with various dermatitis conditions, elevated histamine production by *Clostridium. perfringens* may be the cause of the symptoms related to clostridium dermatitis. Hence, the production of histamine by *C. perfringens* was determined in subjects presenting with clostridium dermatitis.

Three *Clostridium perfringens* isolates from turkeys with clostridial dermatitis were grown in Brain Heart Infusion (BHI) media and assayed for histamine production *in vitro.* As shown in Figure 2, each of the isolates showed an increase in histamine production. Without being limited to a particular mechanism of action, the histamine overproduction mediated by *Clostridium perfringens* as measured and shown in Figure 2 demonstrates a pathway to treatment and/or prophylaxis of clostridial dermatitis in turkeys due to histamine's role in the skin. Moreover, the histamine overproduction mediated by *Clostridium perfringens* is also expected to be a causal agent of enteric diseases as well as adversely impacting immune functioning in the gut through the overgrowth of *C*. *perfringens* and subsequent invasion of the tissues and spreading throughout tissue resulting in *Clostridial* dermatitis in other subjects including avian species, such as chickens.

Using a bacteria either as a probiotic or as a source of enzymes in sufficient amounts, such as from 10⁴ to about 10¹⁴ CFUs of a probiotic and/or from about 10¹ HDU to about 10¹⁰ HDU of an enzyme, the over production of histamine caused by *C. perfringens* will be reduced in the gut. The reduction of histamine through this administration may result in a reduction or loss of symptoms, such as those found in Clostridial dermatitis or clostridial enteric disease.

### Example 2

To establish which strains of bacteria are capable of degrading histamine, an initial experiment was conducted to demonstrate the ability of *Brevibacterium aurantiacum* to degrade histamine. *B. aurantiacum* was isolated from the rind of artisanal cheese and then inoculated into histamine minimalist medium supplemented with 10mM/L of histamine. Following overnight growth, the amount of histamine remaining was quantitated using UHPLC-based methodology as per Villageliu et al. (A microbial endocrinology-based simulated small intestinal medium for the evaluation of neurochemical production by gut microbiota, FEMS Microbiology Ecology, Volume 94, Issue 7, July 2018, fiy096, https://doi.org/10.1093/femsec/fiy096).

As shown in Figure 3, *Brevibacterium aurantiacum* was capable of degrading effectively all of the histamine present within the media. This shows that it is possible to assess the ability of strains of bacteria to degrade histamine from the surrounding environment. Further, use of the identified strains either as a probiotic or as a source of enzymes in, for example, animal feed or through an oral dose may be used to decrease histamine levels in a subject.

### Example 3

While the media used in Examples 1 and 2 show that certain strains of bacteria may alter the amount of histamine within a minimal media using histamine as the only carbon source, a more complex media, with sources of carbon in addition to histamine, better reflect the gut environment. To further investigate different strains of *Clostridium* spp., the ability of *Clostridium septicum* to produce histamine was assessed in a complex medium.

*C. septicum* was isolated from lesions of turkeys suffering from clostridial dermatitis and then inoculated into the rich microbiological BHI medium. Following overnight growth, the amount of histamine produced was quantitated using UHPLC-based methodology as per Villageliu et al. (2018).

As shown in Figure 4 all of the *Clostridium septicum* samples were able to produce histamine in the complex media. This shows that *C*. *septicum* is likely contributing to the increased histamine in the lesions, exacerbating the symptoms of the infection. Hence, it is desirable to prevent the spread of *Clostridium* spp. before they arrive to the dermal layers. As histamine production in the gut can lead to increased permeability and inflammation, preventing the histamine increase in the gut should help prevent the spread of *Clostridium* spp. from the gut to the skin layers. This in turn would reduce the symptoms associated with clostridium dermatitis.

To achieve this reduction of histamine, use of bacteria either as a probiotic or as a source of enzymes to use in, for example, animal feed in a sufficient amount of histamine degrading probiotics, such as 10⁴ to 10¹⁴ CFUs of a probiotic and/or from about 10¹ HDU to about 10¹⁰ HDU of an enzyme, may be administer to a subject. This would result in the decrease in histamine in the gut, thereby preventing the irregularities seen in inflammation and gut permeability by having a high level of histamine present. As such, the administration of a histamine degrading probiotic would decrease or prevent the conditions associated with clostridium dermatitis.

### Example 4 (reference only)

While Experiment 2 shows that some bacterial strains may deplete histamine when it is the only carbon source, histamine may not be the most preferred target for degradation when other carbon sources are available, such as in the gut. Hence, additional strains of bacteria were assayed for their ability to degrade histamine in complex media containing histamine and other carbon sources. *Enterococcus cecorum* isolates were assayed for their ability to degrade histamine in a complex medium. *E. cecorum* were isolated from the intestinal tract of healthy chickens and then inoculated into the rich microbiological medium TSB supplemented with added histamine. Following overnight growth, the amount of histamine remaining was quantitated using UHPLC-based methodology as per Villageliu et al. (2018).

As shown in Figure 5 the *Enterococcus cecorum* strains isolated were capable of degrading histamine to various degrees in the complex media. This indicates that even with additional carbon sources available, isolates of bacteria will degrade histamine. Therefore, the use of bacteria either as a probiotic or as a source of enzymes will degrade histamine in more complex environments, such as the gut. Further, this Example provides a model assay for determining which isolates may degrade more histamine than others, even within the same species of bacteria. This would allow for the use of a probiotic mixture comprised of select community members may be used to degrading histamine in a subject.

### Example 5 (reference only)

To show that this assay may be used across more species, the ability of *Lactobacillus crispatus* to degrade histamine in a complex medium was determined. *L. crispatus* was isolated from the intestinal tract of a healthy pig and then inoculated into the rich microbiological medium MRS supplemented with added histamine. Following overnight growth, the amount of histamine remaining was quantitated using UHPLC-based methodology as per Example 4.

As shown in Figure 6, the *Lactobacillus crispatus* were capable of degrading histamine in the complex media. Therefore, this assay may be used across multiple species of bacteria to determine species and isolates within species that are capable of degrading histamine in the presence of additional carbon sources.

### Example 6

For a microorganism to be safe to administer to a subject, they must be non-pathogenic. One such location in which to identify non-pathogenic strains would be naturally occurring bacteria of the gut. This may be accomplished by feeding a subject a histamine-enriched diet that would force the subject's microbiota to enrich for those gut bacteria that could utilize and degrade the exogenous histamine fed as part of the diet. This method differs from a more traditional *in vitro* screening approach where a whole bank of bacteria is screened for potential histamine degrading activity.

### EXPERIMENTS and METHODOLODY:

### A. Histamine Feeding Trial #1 (reference only)

One day old, unsexed, Cobb chickens were randomly divided into three separate groups. Each group was fed the same basal chicken diet supplemented or not with low amounts of histamine as follows:
1. Group 1 (21 chickens) - Control: no histamine added to feed
2. Group 2 (21 chickens) - Low Histamine: 1mg of histamine dihydrochoride (TCI Chemicals, CAS #56-92-8) was added to 100g of granular feed
3. Group 3 (21 chickens) - High Histamine: 3mg of histamine dihydrochoride was added to 100g of granular feed

All chicken feed was prepared at the Iowa State University feed mill. This amount of histamine was selected to not harm the animals. The histamine was added in as a powder and mixed with the granular chicken feed to obtain a homogeneous mixture. Histamine concentration was verified by measuring on an ultra-high-performance liquid chromatography (UHPLC) instrument as per following section. During the feeding trial, all chickens were fed, and watered *ad libitum* and appropriate enrichment items were placed in the pens. Bodyweights and fecal material were collected weekly starting at 1 week of age. Fecal material was collected by placing the individual chicken in a plastic rat cage for 10-30 minutes until the chicken defecated. One hundred mg of fecal material was collected and placed into a 10ml tube of 1/3^{rd} BHI supplemented with 1 mM histamine for culturing and identification of histamine-degrading bacteria as described below.

Equal numbers of chickens were euthanized at 2, 4, and 5 weeks of age. Chickens were euthanized via CO₂ for 10 minutes with cervical dislocation as a secondary method. After death was confirmed the bifurcated cecum was removed and approximately 100mg of cecal content was immediately extricated and placed into a tube of 1/3 BHI for culturing and identification of histamine-degrading bacteria. Additionally, 100mg of fecal material was collected and placed into a tube for culturing and identification of histamine-degrading bacteria as described below.

### 1. Culturing technique to identify presence of histamine-degrading bacteria:

The culture technique utilized 1/3^{rd} concentration of Brain-Heart Infusion (BHI) medium that was prepared by diluting a one-third volume of full strength BHI prepared according to manufacturer's instructions with 2/3^{rd} volume of phosphate buffered saline (PBS). For example, to achieve 10mls of 1/3^{rd} BHI, 3.33mls of full-strength BHI was added to 6.67mls of PBS+ 2/3 PBS and the final pH adjusted to 6.5. Additionally, the 1/3^{rd} BHI medium was supplemented with 1mM of histamine. After collection of cecal or fecal material from chickens, approximately 100mg of fecal material was placed into the medium containing tube and then repeatedly inverted to achieve a homogenous dispersion of the material into the medium. Tubes were then placed into a microaerophilic environment that was achieved with the use of the Anoxomat gas generating system. All tubes were incubated in a 41.5° C incubator for up to 72 hours with sampling at 48 and 72 hours for UHPLC analysis of histamine content. A decrease in histamine content in those tubes which contained either fecal or cecal material from control tubes that did not contain either fecal or cecal material indicated that histamine degradation occurred due to the presence of histamine degrading bacteria.

### 2. Use of UHPLC for histamine quantitation:

Nine hundred microliters of culture supernatants were collected at designated times as shown in figures from culture tubes and added to 100µL of 2M perchloric acid in a 2mL Eppendorf tube. The tube was then vortexed to mix the samples and then centrifuged at 15,000 rpm for 10 minutes at 4° C to pellet the bacterial contents and provide a cell-free supernatant to be used for histamine quantitation. One-hundred microliters of cell-free supernatant was transferred into a 2mL, 9mm wide amber vial containing 900µL of MD-TM mobile phase.

To analyze on the UHPLC instrument, the quantitation was based on published methodology (Frattini V, Lionetti C. Histamine and histidine determination in tuna fish samples using high-performance liquid chromatography. Derivatization with omicron-phthalaldehyde and fluorescence detection or UV detection of "free" species. J Chromatogr A. 1998;809(1-2):241-5; doi: 10.1016/s0021-9673(98)00157-5; Cicero A, Galluzzo FG, Cammilleri G, Pulvirenti A, Giangrosso G, Macaluso A, et al. Development of a Rapid and Eco-Friendly UHPLC Analytical Method for the Detection of Histamine in Fish Products. Int J Environ Res Public Health. 2020;17(20); doi: 10.3390/ijerph17207453; and Vitali L, Valese AC, Azevedo MS, Gonzaga LV, Costa AC, Piovezan M, et al. Development of a fast and selective separation method to determine histamine in tuna fish samples using capillary zone electrophoresis. Talanta. 2013;106:181-5; doi: 10.1016/j.talanta.2012.12.020). In brief, the amber vials were transferred to the carousel that was cooled to 4° C in order of sequence determined by the sequence created in the Chromeleon 7 instrument program. The samples were analyzed using an Ultimate 3000 UHPLC system including electrochemical detector (ECDRS) and variable wavelength detector (VWD). ECDRS was set to 400mV with a column temperature of 27° C using a C18 Hypersil (150mm length, 3mm diameter, and 2.6µm) column. VWD had two channels with wavelengths set to 210 and 280nm. Flow rate was set to 0.6mL/min using MD-TM from ThermoFisher (10% acetonitrile) and 10% IPA as a rear seal wash. All analyses were completed in the Chromeleon 7 instrument program by analyzing every peak of interest using analytical standards purchased from ThermoFisher Scientific. Results were transferred and calculated in Microsoft Excel and graphed using the Prism 8 statistical program (GraphPad, San Diego, CA).

### 3. Results from Histamine Feeding Trial #1

As shown in Figures 7 and 8, substantial histamine degradation occurred in the cecal cultures from 4-week old and 5-week old chickens from Groups 2 and 3 who were fed increasing amounts of histamine. There was no significant reduction of histamine in cultures from Group 1 chickens that were fed the control, non-histamine, containing diet.

Also, while the addition of the histamine did not result in an increase in blood histamine, there is a slight, non-statistically significant decrease in body weight of the chickens fed the histamine diets, with the higher histamine diet trending toward lower body weights (Figure 21). This trend shows that even non harmful amounts of histamine may be affecting the feeding behavior, causing the chickens to eat less as all cohorts were fed *ab libitum.* As it is detrimental for production animals to not quickly gain weight, it would be a beneficial use of bacteria either as a probiotic or as a source of enzymes to use in animal feed to degrade the histamine in a subject by promoting natural feeding behavior.

### 4. Identification of histamine-degrading bacteria:

From cultures which were found to contain at least 10% histamine degradation as quantitated by UHPLC, colonies were isolated onto selective standard microbiological agar including EMB, MacConkey, CNA and m-Enterococcus selective agars. Individual colonies were then picked off plates and MALDI-TOF technology was used to obtain genus and species identification. The following species were identified as being capable of greater than 10% histamine degradation:
- *Escherichia coli*
- *Enterococcus avium*
- *Enterococcus faecium*
- *Enterococcus* spp.
- *Enterococcus gallinarum*
- *Klebsiella pneumonia*
- *Pseudomonas aeruginosa*

### B. Histamine Feeding Trial #2

A second chicken Histamine Feeding Trial utilized 59, 1-day old and non-sexed Cobb chickens. The groups were as follows:
1. Group 1 (20 chickens) - Control: no histamine added to feed
2. Group 2 (20 chickens) - Low Histamine: 5mg of histamine dihydrochoride (TCI Chemicals, CAS #56-92-8) was added to 100g of granular feed
3. Group 3 (19 chickens) - High Histamine: 10mg of histamine dihydrochloride was added to 100g of granular feed

Fecal matter was collected from pen litters and cultured for the identification of histamine-degrading bacteria as described for the Histamine Feeding Trial #1.

### 1. Results from Histamine Feeding Trial #2 (reference only)

As shown in Figure 9, histamine-degrading bacteria were found in the fecal matter from a Group #3, high histamine chicken. Following isolation of the bacteria onto selective medium and then MALDI-TOF, the identity of the bacteria responsible for the histamine degradation were shown to be comprised of *Enterococcus faecalis, Lactobacillus reuteri,* and *Klebsiella pneumoniae.* There appeared to be cooperativity between each of the bacteria genus/species to aid in the degradation of the histamine.

Similar evidence of cooperativity between different genus/species was observed in cecal matter from a chicken from Group #2, low histamine fed diet. Thawed cecal matter was cultured in 1/3^{rd} BHI supplemented with 1mM histamine. Following the demonstration that substantial histamine degradation occurred as shown in Figure 10, the cultures were sampled in duplicate and then plated onto CNA, m-Enterococcus selective agar, MacConkey and MRS agar media that were supplemented with 2mM histamine. MALDI-TOF analysis of isolated colonies on histamine-containing selective agar resulted in the identification of *Enterococcus faecium, Lactobacillus reuteri,* and *Escherichia coli* in the first duplicate sample. The second duplicate demonstrated the presence, as shown by MALDI-TOF, of *Enterococcus gallinarum, Enterococcus faecium,* and *Escherichia coli.*

Also, like the first feeding trial, as shown in Figure 22, there is a non-statistically significant trend over time for the animals being fed the non-harmful amounts of histamine to present with a lower body weight. At even higher levels, such as levels high enough to be harmful or cause inflammation to increase, this would cause the animals to lose even more weight, both through a decrease in appetite due to histamine induced behavioral changes and through the energy required to activate or increase the activity of the immune system. As such, it would be a beneficial use of bacteria either as a probiotic or as a source of enzymes to use in animal feed to degrade the histamine in a subject by promoting natural feeding behavior and to prevent the activation of the immune system due to high histamine levels.

### Example 7

Examination of banked bacterial strains such as *Brevibacterium* spp. were performed for their ability to degrade histamine as a rapid and cost-effective means to identify potential histamine degrading bacteria that could be utilized as a histamine-degrading probiotic. Also, bacteria identified in the *in vivo* experiments above, were further quantified for their ability to degrade histamine.

### EXPERIMENTS and METHODOLODY:

### A. Brevibacterium

*Brevibacterium* spp. are known to be capable of degrading histamine. As such, *Brevibacterium* isolates were evaluated in a histamine minimal medium (HMM) and a histamine-supplemented PBS broth. The HMM basal medium was composed of 6.8g of potassium phosphate, 1.0g of ammonium sulfate and 0.125g of magnesium sulfate heptahydrate in 500ml of deionized water that was sterilized by autoclaving at standard conditions of 121°C for 20 minutes. Additionally, a stock vitamin solution in 10ml of deionized water was prepared which consisted of 3mg of biotin, 12mg of thiamine hydrochloride and 1.5mg of calcium pantothenate. Prior to use the vitamin stock solution was filter sterilized by passage through a 0.22µm syringe filter. Also, a stock iron solution was prepared which consisted of 25mg of iron (III) sulfate heptahydrate dissolved in 5ml of deionized water and then filter sterilized by passage through a 0.22µm syringe filter. To assemble the complete HMM, to the 500ml of autoclaved medium, 500µL of the sterile vitamin mix and 50µL of the sterile iron solution was added and thoroughly mixed and the final pH adjusted to 6.5. The PBS basal medium was obtained from Life Technologies (Gibco product #20012027). The HMM and PBS media were further sterilely supplemented copper sulfate (10µM, final concentration), zinc sulfate (10µM, final concentration), and pyridoxal phosphate (20µg/ml, final concentration). The supplemented HMM and PBS media were further supplemented with histamine at a final concentration of 10mM and then inoculated with *Brevibacterium* spp. strains and incubated at 31° C for 96 hours. Culture supernatants were then obtained and processed for histamine quantitation as described above.

Additionally, 1/3^{rd} dilution of full-strength BHI was prepared as a growth medium as described above. Also, a 1/3^{rd} dilution of full-strength Tryptic Soy Broth (TSB) was similarly prepared. Both media, in addition to full strength BHI and TSB, were further supplemented with histamine (1mM, final concentration), copper sulfate (10µM, final concentration), zinc sulfate (10µM, final concentration), and pyridoxal phosphate (20µg/ml, final concentration). A Brevibacterium strain plated onto Tryptic Soy agar and grown aerobically at 30°C overnight was then inoculated into the growth media using a 10µL inoculation loop. Tubes were then cultured aerobically at 30°C for 72 hours on a shaker platform. Culture tubes were sampled at regular time intervals for determination of extent of histamine degradation.

### 1. Results from Brevibacterium evaluation

As shown in Figure 11, three different isolates of *Brevibacterium* spp., namely ML1292, *Brevibacterium* spp.; ML1293, *Brevibacterium* spp.; and ML1233, *Brevibacterium* spp. were able to substantially decrease the amount of histamine in two different types of histamine-supplemented minimal media over a 96 hours' time course.

As shown in Figure 12, ML1293, *Brevibacterium* spp. was able to substantially decrease the amount of histamine in two different types of histamine-supplemented minimal media over a 72 hours' time course.

### 2. Results from Brevibacterium isolated from poultry feces collected in poultry litter

Fecal containing poultry litter from 4-6 week unsexed, Cobb chickens was collected and Brevibacterium isolated and then tested for their ability to degrade histamine using the 1/3^{rd} BHI and TSB formulations as described above for the *Brevibacterium* methodology.

As shown in Figure 18, *Brevibacterium* spp. isolated from poultry fecal matter in the poultry litter and identified by MALDI-TOF as well as Sanger sequencing, specifically ML1197, *Brevibacterium aureum*; ML1205, *Brevibacterium sediminis*; and ML1268, *Brevibacterium epidermis* were able to substantially decrease the amount of histamine in 1/3^{rd} BHI-supplemented medium over a 72 hours' time course.

As shown in Figure 19, *Brevibacterium* spp. isolated from poultry fecal matter in the poultry litter, specifically ML1197, *Brevibacterium aureum*; ML1205, *Brevibacterium sediminis;* and ML1268, *Brevibacterium epidermis,* and ML1203, *Brevibacterium sediminis* decrease the amount of histamine in 1/3^{rd} TSB-supplemented medium over a 72 hours' time course.

### B. Use of histamine-degrading bacteria from Histamine Feeding Trial #1:

Overnight cultures from Histamine Feeding Trial #1 that were stored at 4°C were inoculated onto 1/3^{rd} BHI supplemented with 10mM histamine agar plates using 10µL inoculation loop. Following growth, a 10µL inoculation loopful of bacteria was harvested for subsequent inoculation and dispersal into 3ml of sterile PBS. One-hundred microliters of the bacterial culture solution was then inoculated into 1/3^{rd} BHI, HMM, and Brevi Histamine broth (BHB) supplemented with histamine ranging in concertation from 1mM to 30mM. All growth experiments were conducted in a microaerophilic environment maintained at 41.5° C. Culture supernatants were then obtained and processed for histamine quantitation as described above.

### 1. Results from use of histamine-degrading bacteria from Histamine Feeding Trial #1 (reference only):

As shown in Figure 13, *Enterococcus* spp. isolated from individual chickens from Group #3 fed a histamine containing diet were able to substantially decrease the amount of histamine in 1/3^{rd} BHI-supplemented histamine medium over a 120 hours' time course. The medium initially contained 10mM of histamine at the start of the incubation period.

Similarly, as shown in Figure 14, *Enterococcus* spp. isolated from individual chickens from Group #3 fed a histamine containing diet were able to substantially decrease the amount of histamine in 1/3^{rd} BHI-supplemented histamine medium as well as in HMM-supplemented histamine medium over a 72 hours' time course. The media initially contained 10mM of histamine at the start of the incubation period.

### C. Ability of Pseudomonas spp. to degrade histamine (reference only)

As data from the Histamine Feeding Trial #1 had identified *Pseudomonas aeruginosa* as a strain capable of degrading histamine, further experiments were conducted to examine if this ability was solely restricted to *Pseudomonas* spp. isolated from poultry. *P. aeruginosa* that had been previously isolated from dogs at different anatomical sites and a *Pseudomonas taetrolens* isolate from a pig were inoculated into HMM supplemented with 10mM histamine at the start of the incubation period. As shown in Figure 20, *Pseudomonas* spp. strains from all animal species substantially decreased the amount of histamine following 24 hours incubation.

### D. Ability of yeast to degrade histamine (reference only)

The yeast *Candida krusei* is a potential probiotic that was examined for its ability to degrade histamine. Following overnight aerobic growth in BHI at 41.5°, it was inoculated into full strength BHI broth supplemented with 1mM histamine and placed on a rocking platform set with a speed of 20RPM and 8° tilt. At the end of the incubation period culture supernatants were then obtained and processed for histamine quantitation as described above.

### 1. Results from use of histamine-degrading yeast:

As shown in Figure 15, *Candida krusei* was capable of substantially reducing the amount of the amount of histamine in BHI-supplemented histamine medium over a 72 hours' time course. The medium initially contained 1 mM of histamine at the start of the incubation period.

These results also show that it may be preferrable to knock down or knock out the function of histidine decarboxylase in growth conditions that provide carbon sources other than histamine. As shown in Figure 15, the level of histamine raises initially, but decreases over time.

### Example 8 (reference only)

The ability of amine oxidase (copper containing) from animal sources to degrade histamine has been well established in the scientific literature for decades. Further, amine oxidase (copper containing) exists in plants and has been shown to be of potential benefit to human disease.

### EXPERIMENTS and METHODOLODY:

### A. Diamine production by pea shoots (reference only)

Certified organic dried green pea sprouting seeds (*Lathyrus sativus*) were grown in a soil-free seed sprouting tray in the dark as per published studies. Sprouts were harvested at 10 days and immediately frozen at -20°C freezer pending subsequent processing for diamine oxidase. Following thawing, the shoots were ground in a pre-chilled mortar and pestle to obtain a fine pulp that was then transferred to a 15ml conical tube and approximately 11mls of pre-chilled PBS, pH 7 was then added to suspend the pulp. All tubes were placed in a rotating rack at 4°C and spun end over end at low speed for 30 minutes. After rotation, the tubes are transferred to a centrifuge and centrifuged at 2000 rpm at 4°C for 10 minutes to pellet any undissolved material. The supernatant was then removed, and syringe filtered using a 0.22µm filter to ensure there were no possible contaminants or large particles. The supernatant was used to inoculate histamine supplemented PBS, LB broth, and BHI broth at a final v/v ratio between 10 and 1%. The amount of histamine supplementation of all broth media was 1mM. Tubes were next incubated aerobically at 37°C for up to 24 hours on a rocker with a 15-degree tilt at 20RPM. At designated time intervals as shown in the figures, an aliquot from each tube was removed and analyzed for extent of histamine degradation as described above for bacterial cultures.

### 1. Results from use of pea shoots to produce sufficient diamine oxidase to degrade histamine:

As shown in Figure 16, a crude extract of amine oxidase (copper containing) from 10-day green pea shoots grown in the dark contained significant amounts of amine oxidase (copper containing) as evidenced by the ability of the extract to decrease histamine content in a number of the indicated histamine-supplemented media.

As shown in Figure 17, a crude extract of amine oxidase (copper containing) from 10-day green pea shoots grown in the dark contained significant amounts of amine oxidase (copper containing) that exhibited dose-dependency as evidenced by the ability of greater amounts of the extract to decrease greater amounts of histamine content over a 24-hour time period.

Therefore, plants, in addition to microorganisms, may be a source of enzymes for histamine breakdown.

### Example 9 (reference only)

Examples 1-8 show that it is possible to identify safe probiotic microorganism which may be used either as a probiotic or as a source of enzymes to degrade histamine levels of the gut. As there is a link between neurotransmitters and other compounds of the gut and the rest of the body, for example, the gut-brain axis, this degradation of histamine would be expected to have system effects.

These effects would be mediated through the H₁ through H₄ receptors as discussed above. For example, it would be predicted that the loss of histamine in the gut would lead to an increase in feeding behavior as both the H₁ and H₃ receptors would receive less signal. The decrease of histamine would also be predicted to lower allergic reactions as histamine provides a powerful pro-inflammatory signal through H₁, H₂, and H₄ receptors. Further, as cancer cells express histamine receptors and use histamine to regulate proliferation and angiogenesis, the loss of histamine may be used to treat neoplastic diseases. Therefore, diseases and symptoms throughout a subject may be treated by a probiotic which degrades histamine.

More locally, the decrease in histamine would decrease the symptoms caused by pathogens which rely on histamine as a virulence factor, such as *Clostridium* spp., as the histamine would be degraded before it could cause disease. Further, it would also reduce gut inflammation as the pro-inflammatory signal would be reduced. This reduction in inflammation in the gut would be predicted to lower the inflammation during necrotizing enterocolitis and may prevent the gut from becoming permeable, preventing the spillage of stool into the body cavity. Therefore, bacteria in the gut may be regulated and local gut heath may be improved by probiotics which degrade histamine.

To achieve this, a therapeutically effective amount of probiotic, such as between 10⁴ to about 10¹⁴ CFUs of histamine degrading bacteria or fungi, may be administered to the subject. Preferably, the probiotic may be administered neat, coated or encapsulated and administered in a feed source and/or as a synbiotic. Instead of bacterium or fungi, or in addition to, the compositions may contain from about 10¹ HDU to about 10¹⁰ HDU enzyme. The administered probiotic, synbiotic, and/or enzyme would then decrease histamine in the gut and treating the symptoms of related histamine conditions.

Further, by administration of the composition prior to the formation of the histamine related conditions, the probiotic may act as a prophylactic. For example, a probiotic, synbiotic, and/or enzyme may be administered prior to a high histamine meal, such as fermented foods or fish meal, to prevent an acute rise in histamine. Or, if the diet will be maintained, the composition could be provided with the feed to prevent a chromic increase of histamine in the gut, preventing excessive inflammation, gut permeability, or the buildup of pathogenic microorganisms which rely on histamine from arising in a subject.

Further, as shown in Example 3, as C. *septicum* was isolated from lesions, applying the compositions topically would decrease the histamine overproduction within the lesion.

Therefore, use of bacteria or fungi either as a probiotic or as a source of enzymes (in addition to plants) to use for oral or topical administration may be used to treat and/or prevent clostridial dermatitis, clostridial enteric disease, pathogens for which histamine is a virulence factor, gut inflammation, necrotizing enterocolitis, neoplastic diseases, behavior, and/or dysregulated histamine production.

The invention is defined by the appended claims.

## Claims

1. A probiotic composition for use in a method of preventing and/or treating clostridial dermatitis and/or clostridial enteric disease in a subject comprising:
histamine-degrading bacteria, wherein the bacteria comprise *Brevibacterium aureum, Brevibacterium sediminis,* and/or *Brevibacterium epidermidis*;
wherein said composition controls histamine production from a commensal microbiota in the subject.

2. A non-therapeutic method of increasing feeding efficiency of a subject comprising administering:
a probiotic composition comprising histamine-degrading bacteria, wherein the bacteria comprise *Brevibacterium aureum, Brevibacterium sediminis,* and/or *Brevibacterium epidermidis;*
wherein said composition controls histamine production in the subject.

3. The composition for use of claim 1, wherein the subject is a farm production animal of an avian species that is one or more of a chicken, duck, goose, turkey, guinea fowl, ostrich, pigeon, quail, and pheasant.

4. The composition for use of any one of claims 1 or 3, wherein the subject is a companion animal, an aquaculture species or a human.

5. The composition for use of claim 1, wherein the commensal microbiota comprises *Clostridium* spp., *Salmonella* spp., *Escherichia* spp., *Proteus* spp., *Enterobacter* spp., *Klebsiella* spp., *Morganella* spp., and/or *Lactobacillus* spp.

6. The composition for use of claim 5, wherein the probiotic composition is administered to a subject with a *Clostridial* infection.

7. The composition for use of claim 5, wherein the probiotic composition is administered to a subject as prophylaxis.

8. The composition for use of any one of claims 1 or 3 to 7, wherein the probiotic composition is a live, non-pathogenic microorganism, a spore that is germinated into a histamine-degrader in the subject, or a mash incorporating a microorganism therein.

9. The composition for use of any one of claims 1 or 3 to 8, wherein the probiotic composition is neat, coated or encapsulated and administered in a feed source.

10. The composition for use of any one of claims 1 or 3 to 9, wherein the probiotic composition contains a therapeutically effective amount of the strain(s) from about 10⁴ CFU to about 10¹⁴ CFU.

11. The composition for use of any one of claims 1, 3 to 8 or 10, wherein the administration is oral or topical.

## Patentansprüche

1. Probiotische Zusammensetzung zur Verwendung in einem Verfahren zur Vorbeugung und/oder Behandlung von clostridialer Dermatitis und/oder clostridialer enterischer Erkrankung bei einem Subjekt, umfassend:
Histamin abbauende Bakterien, wobei die Bakterien *Brevibacterium aureum, Brevibacterium sediminis* und/oder *Brevibacterium epidermidis* umfassen;
wobei die Zusammensetzung die Histaminproduktion einer kommensalen Mikroflora in dem Subjekt steuert.

2. Nicht therapeutisches Verfahren zur Erhöhung der Fütterungseffizienz eines Subjekts, umfassend Verabreichen von:
einer probiotischen Zusammensetzung, umfassend Histamin abbauende Bakterien, wobei die Bakterien *Brevibacterium aureum, Brevibacterium sediminis* und/oder *Brevibacterium epidermidis* umfassen;
wobei die Zusammensetzung die Histaminproduktion in dem Subjekt steuert.

3. Zusammensetzung zur Verwendung nach Anspruch 1, wobei es sich bei dem Subjekt um ein Tier einer Vogelart für die landwirtschaftliche Produktion handelt, das eines oder mehrere von Huhn, Ente, Gans, Truthahn, Perlhuhn, Strauß, Taube, Wachtel und Fasan ist.

4. Zusammensetzung zur Verwendung nach einem der Ansprüche 1 oder 3, wobei es sich bei dem Subjekt um ein Haustier, eine Aquakultur-Art oder einen Menschen handelt.

5. Zusammensetzung zur Verwendung nach Anspruch 1, wobei die kommensale Mikroflora *Clostridium* spp., *Salmonella* spp., *Escherichia* spp., *Proteus* spp., *Enterobacter* spp., *Klebsiella* spp., *Morganella* spp. und/oder *Lactobacillus* spp. umfasst.

6. Zusammensetzung zur Verwendung nach Anspruch 5, wobei die probiotische Zusammensetzung einem Subjekt mit einer clostridialen Infektion verabreicht wird.

7. Zusammensetzung zur Verwendung nach Anspruch 5, wobei die probiotische Zusammensetzung einem Subjekt als Prophylaxe verabreicht wird.

8. Zusammensetzung zur Verwendung nach einem der Ansprüche 1 oder 3 bis 7, wobei es sich bei der probiotischen Zusammensetzung um einen lebenden, nicht pathogenen Mikroorganismus, eine Spore, die in dem Subjekt zu einem Histamin abbauenden Organismus auskeimt, oder eine Maische, die einen Mikroorganismus darin einschließt, handelt.

9. Zusammensetzung zur Verwendung nach einem der Ansprüche 1 oder 3 bis 8, wobei die probiotische Zusammensetzung rein, beschichtet oder verkapselt ist und in einer Futterquelle verabreicht wird.

10. Zusammensetzung zur Verwendung nach einem der Ansprüche 1 oder 3 bis 9, wobei die probiotische Zusammensetzung eine therapeutisch wirksame Menge des Stammes/der Stämme von etwa 10⁴ KBE bis etwa 10¹⁴ KBE enthält.

11. Zusammensetzung zur Verwendung nach einem der Ansprüche 1, 3 bis 8 oder 10, wobei die Verabreichung oral oder topisch erfolgt.

## Revendications

1. Composition probiotique destinée à être utilisée dans un procédé de prévention et/ou de traitement de la dermatite clostridienne et/ou de la maladie entérique clostridienne chez un sujet comprenant :
des bactéries dégradant l'histamine, dans laquelle les bactéries comprennent *Brevibacterium aureum, Brevibacterium sediminis* et/ou *Brevibacterium epidermidis ;*
dans laquelle ladite composition contrôle la production d'histamine à partir d'un microbiote commensal chez le sujet.

2. Procédé non thérapeutique d'amélioration de l'efficacité alimentaire d'un sujet, comprenant l'administration :
d'une composition probiotique comprenant des bactéries dégradant l'histamine, dans laquelle les bactéries comprennent *Brevibacterium aureum, Brevibacterium sediminis* et/ou *Brevibacterium epidermidis ;*
dans laquelle ladite composition contrôle la production d'histamine chez le sujet.

3. Composition destinée à être utilisée selon la revendication 1, dans laquelle le sujet est un animal de production agricole d'une espèce aviaire qui est l'une ou plusieurs parmi un poulet, un canard, une oie, une dinde, une pintade, une autruche, un pigeon, une caille et un faisan.

4. Composition destinée à être utilisée selon l'une quelconque des revendications 1 ou 3, dans laquelle le sujet est un animal de compagnie, une espèce aquacole ou un être humain.

5. Composition destinée à être utilisée selon la revendication 1, dans laquelle le microbiote commensal comprend *Clostridium* spp., *Salmonella* spp., *Escherichia* spp., *Proteus* spp., *Enterobacter* spp., *Klebsiella* spp., *Morganella* spp. et/ou *Lactobacillus* spp.

6. Composition destinée à être utilisée selon la revendication 5, dans laquelle la composition probiotique est administrée à un sujet atteint d'une infection *clostridienne.*

7. Composition destinée à être utilisée selon la revendication 5, dans laquelle la composition probiotique est administrée à un sujet à titre prophylactique.

8. Composition destinée à être utilisée selon l'une quelconque des revendications 1 ou 3 à 7, dans laquelle la composition probiotique est un micro-organisme vivant non pathogène, une spore qui est germée en un micro-organisme dégradant l'histamine dans le sujet, ou une préparation alimentaire incorporant un micro-organisme.

9. Composition destinée à être utilisée selon l'une quelconque des revendications 1 ou 3 à 8, dans laquelle la composition probiotique est pure, enrobée ou encapsulée et administrée dans une source d'alimentation.

10. Composition destinée à être utilisée selon l'une quelconque des revendications 1 ou 3 à 9, dans laquelle la composition probiotique contient une quantité thérapeutiquement efficace de la ou des souches d'environ 10⁴ UFC à environ 10¹⁴ UFC.

11. Composition destinée à être utilisée selon l'une quelconque des revendications 1, 3 à 8 ou 10, dans laquelle l'administration est orale ou topique.
